# EUROPÄISCHE PATENTSCHRIFT

(11) **EP 1 285 943 B1**
(45) Veröffentlichungstag und Bekanntmachung des Hinweises auf die Patenterteilung: **19.10.2005**
(21) Anmeldenummer: 02017975.0
(22) Anmeldetag: 10.08.2002
(51) Int. Cl.: C08G 77/388

(54) **UV-Licht absorbierende quartäre Polysiloxane**
Uv-absorbing quaternary polysiloxanes
Polysiloxanes quaternaires absorbant la lumière ultraviolette

(30) Priorität: 23.08.2001 DE 10141356
(43) Veröffentlichungstag der Anmeldung: 26.02.2003
(73) Patentinhaber: Goldschmidt GmbH, 45127 Essen (DE)
(72) Erfinder: Fender, Michael, 36103 Flieden (DE); Krakenberg, Manfred, 45307 Essen (DE); Leidreiter, Holger, Dr., 45529 Hattingen (DE); Oestreich, Sascha, Dr., 45279 Essen (DE)

(56) Entgegenhaltungen:
- EP-A- 0 530 974
- US-A- 4 891 166

## Beschreibung

Die Erfindung betrifft UV-Licht absorbierende quartäre Polysiloxane, Verfahren zu deren Herstellung und deren Verwendung in Formulierungen zur Textilpflege und Kosmetikzubereitungen.

Es ist bekannt, dass UV-Licht der Wellenlängen von 200 bis 400 nm für das Ausbleichen und die Schädigung von Textilien, synthetischen Fasern und natürlichen Fasern (z.B. Wolle, Baumwolle und Haare) verantwortlich ist.

Aus diesem Grund besteht ein wachsender Bedarf an Verbindungen, die die Textilien, synthetischen Fasern und natürlichen Fasern vor der UV-Strahlung abschirmen oder eine Kontrolle über den Grad der Schädigung erlauben.

Es ist daher wünschenswert, Verbindungen bereitzustellen, die eine Kontrolle über die UV-Strahlung ausüben können, der die Textilien bzw. die synthetischen oder natürlichen Fasern ausgesetzt werden.

Aus der Literatur ist eine große Anzahl von Verbindungen bekannt, die für den UV-Lichtschutz von Fasern, Farbstoffen und Pigmenten eingesetzt werden. Solche Verbindungen werden typischerweise direkt bei der Herstellung der Faser eingesetzt.

Diese Verbindungen zeigen aber keinen pflegenden bzw. weichmachenden Effekt. Zusätzlich fehlt ihnen oft die Haftung auf der Faseroberfläche, so dass schon nach wenigen Waschvorgängen der UV-Schutz auf der Faser verlorengeht.

Es ist daher wünschenswert, Verbindungen bereitzustellen, die einen pflegenden Effekt auf natürliche oder synthetische Fasern ausüben, eine hohe Substantivität auf der Faser zeigen und zusätzlich einen Schutz vor Schädigungen durch mechanische und/oder optische (z.B. UV-Licht) Einflüsse bieten.

Um auch nach mehrmaliger Wäsche einen hinreichenden UV-Schutz bereitstellen zu können, sollten die Verbindungen auch in Weichspülformulierungen einarbeitbar sein und während des Weichspülvorgangs auf die Faser aufziehen.

Quartäre Polysiloxane sind ebenfalls aus der Literatur bekannt und zum Beispiel in den Patenten EP-A-0 282 720 und DE-A-37 19 086 beschrieben. Solche Verbindungen sind besonders für ihre konditionierenden Eigenschaften in der Haarkosmetik und für ihre weichmachenden und pflegenden Effekte in der Textilbehandlung bekannt. Verbindungen dieser Art werden weiterhin eingesetzt, um die Elastizität bzw. die Reißfestigkeit von Textilien zu erhöhen und deren Falten- bzw. Knitterbildung zu verringern und/oder das spätere Bügeln zu erleichtern ("easy ironing") (WO-01/25385, WO-01/25382, WO-01/25381, WO-01/25380, WO-99/55953). Verbindungen, wie sie in den Patenten EP-282 720 und DE-37 19 086 beschrieben sind, zeigen jedoch keinen Schutz vor dem schädlichen Einfluss der UV-Strahlung.

Nachteilig gemäß dem Stand der Technik ist es daher, dass jeweils spezielle Verbindungen bereitgestellt werden müssen, um die oben beschriebenen Effekte zu erzielen.

Die komplexe Aufgabe der vorliegenden Erfindung bestand nun darin, Verbindungen bereitzustellen, die universell angewandt werden können, gleichzeitig einen glättenden, pflegenden und weichmachenden Effekt auf natürliche oder synthetische Fasern ausüben, eine hohe Substantivität auf den natürlichen oder synthetischen Fasern (z.B. Wolle, Baumwolle oder Haare) oder anderen Oberflächen (z.B. der Haut) zeigen und zusätzlich Schädigungen durch mechanische und/oder optische Einflüsse (z.B. UV-Licht) vermindern.

Überraschenderweise wurde nun gefunden, dass die vorgenannte Aufgabe gelöst wird durch UV-Licht absorbierende quartäre Polysiloxane.

Gegenstand der Erfindung sind daher UV-Licht absorbierende quartäre Polysiloxane der allgemeinen Formel (I) 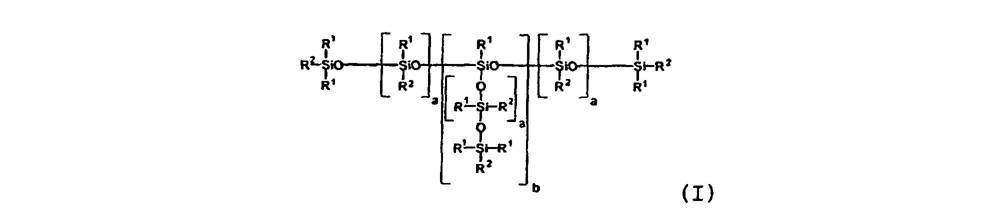 worin die Reste
- R¹: gleich oder verschieden sind und jeweils niedere Alkylreste mit 1 bis 4 Kohlenstoffatomen oder Phenylreste bedeuten,
- R²: zu einem Teil die Bedeutung der Reste R¹ haben können und die übrigen Reste R² Reste der Formel (Ia)

R² = -M-Z⁺ A⁻ (Ia)

sind, mit der Maßgabe, dass im durchschnittlichen Molekül mindestens ein Rest R² ein Rest der Formel -M-Z⁺ A⁻ ist,
worin
Z ein Rest der Formel (Ib) 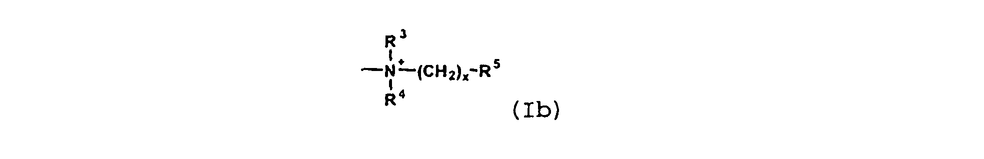 ist,
R³, R⁴ Alkylreste mit 1 bis 22 Kohlenstoffatomen oder Alkenylreste mit 2 bis 22 Kohlenstoffatomen, worin die Alkyl- oder Alkenylreste Hydroxylgruppen aufweisen können,
R⁵ ein einwertiger, für die UV-Absorption verantwortlicher Chromophorrest, der Formel (Ic) 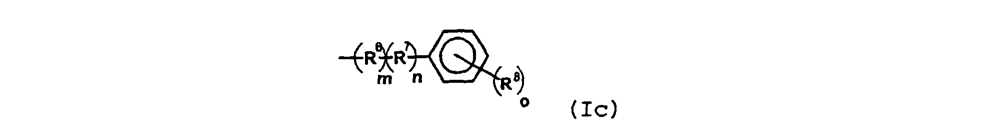 worin
R⁶ 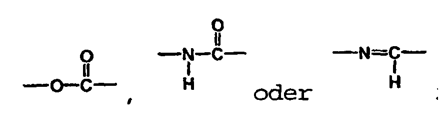 ist,
R⁷ -CH=CH- ist,
R⁸ gleich oder verschieden sind und jeweils Wasserstoff, Alkyl-, Haloalkyl-, Halogen-, Phenyl-, Hydroxyl-, Alkoxy-, Amino-, Alkylamino-, Dialkylamino-, Di(hydroxyalkyl)amino- oder Di(polyalkoxy)aminoreste bedeuten,
m = 0 oder 1,
n = 0 oder 1,
o = 0 bis 5,
x = 0 bis 6,
M ein zweiwertiger Kohlenwasserstoffrest mit mindestens 4 Kohlenstoffatomen ist, der eine Hydroxylgruppe aufweist und der durch ein oder mehrere Sauerstoffatome unterbrochen sein kann, wobei das N-Atom des Restes Z mit dem Rest M über das zur C-OH-Gruppe im Rest M benachbarte Kohlenstoffatom verbunden ist,
A⁻ ein anorganisches oder organisches Anion, das von einer üblichen physiologisch verträglichen Säure HA herrührt, ist,
- a: xunabhängig voneinander einen Wert von 1 bis 200 und
- b: einen Wert von 0 bis 10 hat.

Ein weiterer Gegenstand der vorliegenden Erfindung besteht in der Herstellung der erfindungsgemäßen Verbindungen. Das erfindungsgemäße Verfahren ist dadurch gekennzeichnet, dass man Verbindungen der allgemeinen Formel (II) 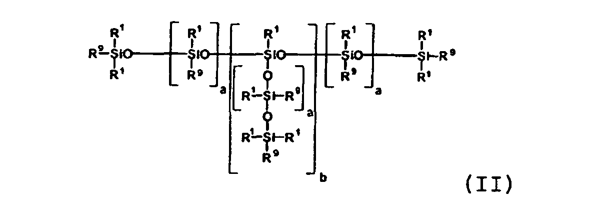 worin die Reste
- R¹: gleich oder verschieden sind und jeweils niedere Alkylreste mit 1 bis 4 Kohlenstoffatomen oder Phenylreste bedeuten,
- R⁹: zu einem Teil die Bedeutung der Reste R¹ haben können und die übrigen Reste R⁹ einwertige Reste sind, welche der Struktur des Restes M entsprechen, wobei der Rest R⁹ anstelle der Verknüpfung mit Z und der Hydroxylgruppe eine Epoxidgruppe aufweist,
mit der Maßgabe, dass im durchschnittlichen Molekül mindestens ein Rest R⁹ ein einwertiger Rest ist, welcher der Struktur des Restes M entspricht, wobei der Rest R⁹ anstelle der Verknüpfung mit Z und der Hydroxylgruppe eine Epoxidgruppe aufweist,
a unabhängig voneinander einen Wert von 1 bis 200 und
b einen Wert von 0 bis 10 hat, mit tertiären Aminen der allgemeinen Formel (IIb) 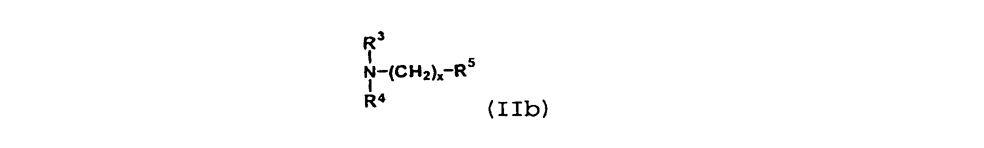 worin
R³, R⁴ Alkylreste mit 1 bis 22 Kohlenstoffatomen oder Alkenylreste mit 2 bis 22 Kohlenstoffatomen, worin die Alkyl- oder Alkenylreste Hydroxylgruppen aufweisen können,
R⁵ ein einwertiger, für die UV-Absorption verantwortlicher Chromophorrest, der Formel (Ic) 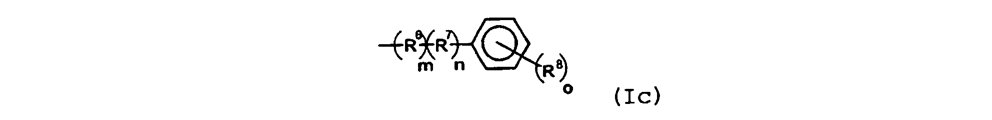 worin
R⁶ 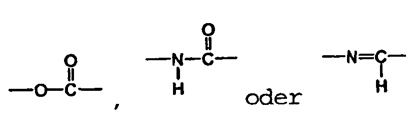 ist,
R⁷ -CH=CH- ist,
R⁸ gleich oder verschieden sind und jeweils Wasserstoff, Alkyl-, Haloalkyl-, Halogen-, Phenyl-, Hydroxyl-, Alkoxy-, Amino-, Alkylamino-, Dialkylamino-, Di(hydroxyalkyl)amino- oder Di(polyalkoxy)aminoreste bedeuten,
m = 0 oder 1
n = 0 oder 1,
o = 0 bis 5,
x = 0 bis 6,
in an sich bekannter Weise in solchen Mengenverhältnissen umsetzt, dass jeder Epoxidgruppe mindestens eine tertiäre Aminogruppe entspricht, und man die Umsetzung in Gegenwart eines üblichen physiologisch verträglichen organischen oder anorganischen Säureequivalentes HA, bezogen auf zu quaternisierendes Stickstoffatom, und bei Temperaturen von 40 bis 120 °C durchführt.

Bevorzugte Beispiele für den Rest R¹ sind niedere Alkylreste mit 1 bis 4 Kohlenstoffatomen oder der Phenylrest, wie der Methyl-, Ethyl-, n-Propyl-, iso-Propyl- oder n-Butylrest, iso-Butylrest.

Bevorzugte Beispiele für den Rest M, einen zweiwertigen Kohlenwasserstoffrest mit mindestens 4 Kohlenstoffatomen, der eine Hydroxylgruppe aufweist und der durch ein oder mehrere Sauerstoffatome unterbrochen sein kann, wobei das N-Atom des Restes Z mit dem Rest M über das zur C-OH-Gruppe im Rest M benachbarte Kohlenstoffatom verbunden ist, sind 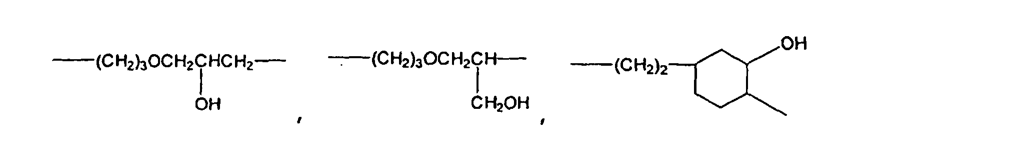 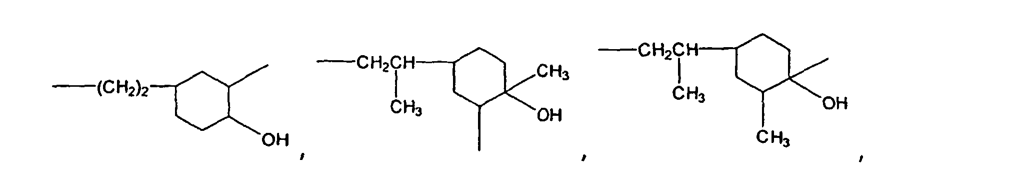 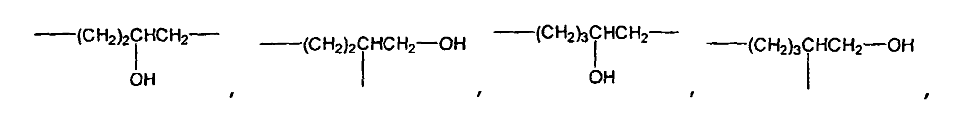 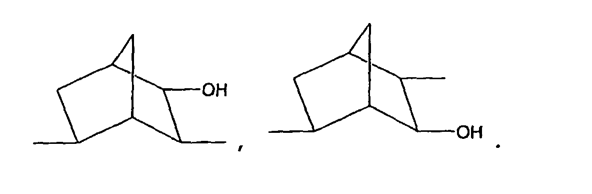 Innerhalb der erfindungsgemäßen Verbindungen können die Reste Z gleiche oder unterschiedliche Bedeutung haben.

Bevorzugte Beispiele für den Rest Z sind

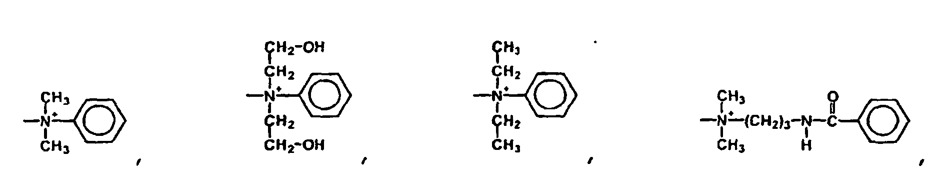

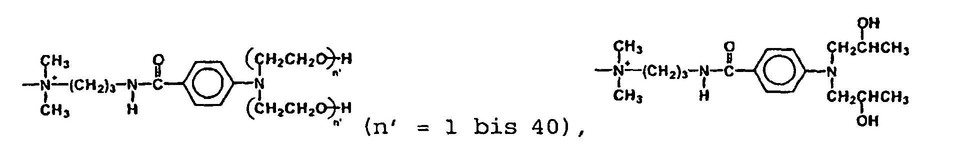

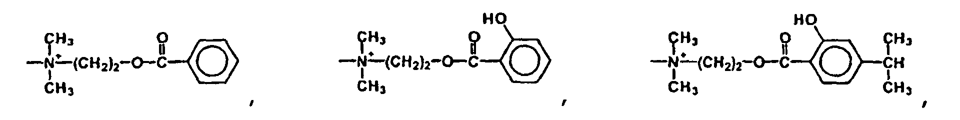

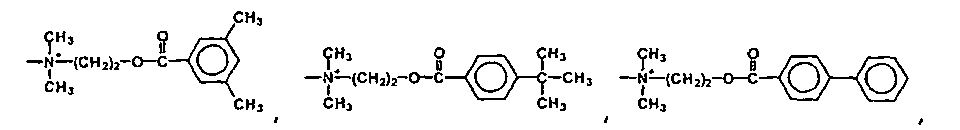

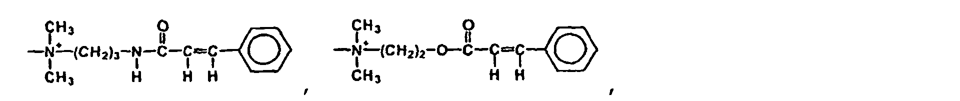

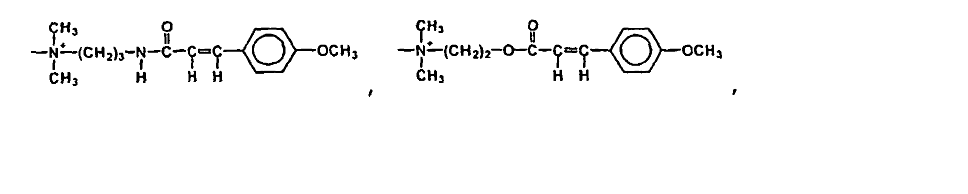

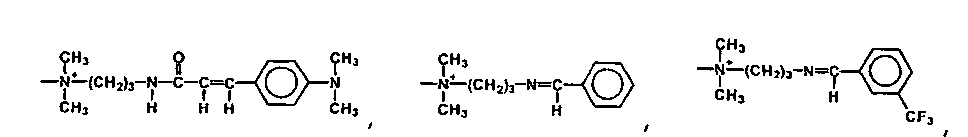

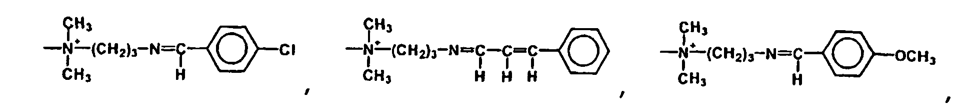

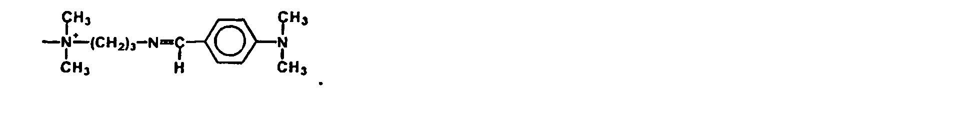

Bevorzugte Beispiele für A⁻, ein anorganisches oder organisches Anion, das von einer üblichen physiologisch verträglichen Säure HA herrührt, sind Acetat-, Chlorid-, Bromid-, Hydrogensulfat-, Sulfat-, Methosulfat-, Ethosulfat-, Citrat-, Tartrat- und Lactationen, sowie Anionen aromatischer Säuren, wie die Anionen der p-Toluolsulfonsäure, Benzoesäure, Salicylsäure, Zimtsäure, 4-Methoxyzimtsäure, 4-Aminobenzoesäure, 4-Bis(hydroxypropyl)-aminobenzoesäure, 4-Bis(polyethoxy)aminobenzoesäure, 4-Dimethylaminobenzoesäure, 3-Imidazol-4-yl-acrylsäure, 2-Phenylbenzimidazol-5-sulfonsäure, 3,3'-(1,4-Phenylendimethin)-bis-(7,7-dimethyl-2-oxo-bicyclo-[2.2.1]heptan-1-methansulfonsäure), 2-Hydroxy-4-methoxybenzophenon-5-sulfonsäure und 3-(4'-Sulfo)-benzyliden-bornan-2-on.

Es ist dem Fachmann geläufig, dass die Verbindungen in Form eines Gemisches mit einer im wesentlichen durch statistische Gesetze geregelten Verteilung vorliegen. Die Werte für die Indices a und b stellen deshalb Mittelwerte dar.

Bevorzugte Beispiele für UV-Licht absorbierende quartäre Polysiloxane, sind Verbindungen der Formeln

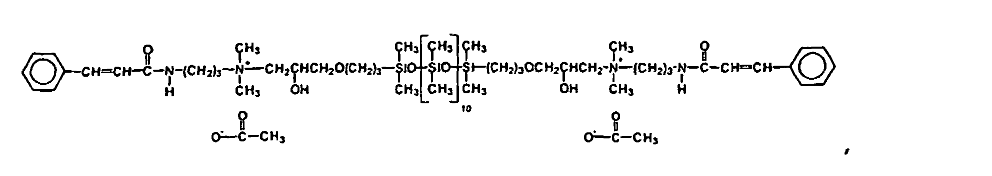

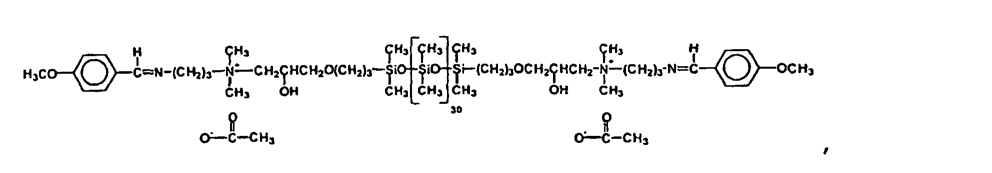

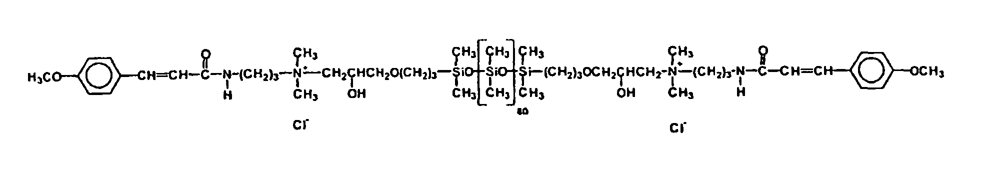

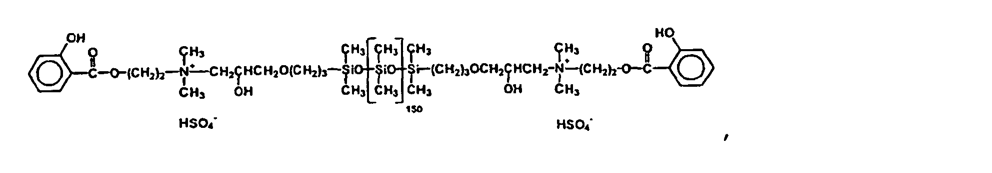

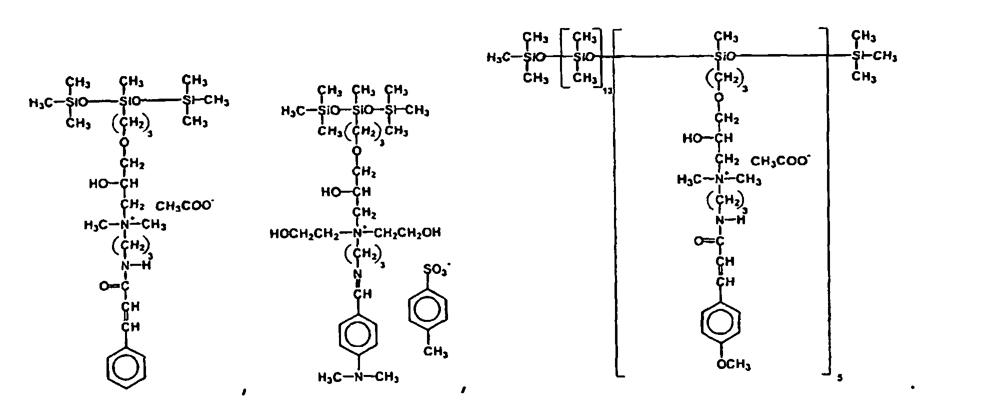

Verfahren zur Herstellung der erfindungsgemäßen Verbindungen und die Eigenschaften dieser Verbindungen sind in den folgenden Beispielen näher beschrieben:

### Beispiel 1:

Herstellung eines erfindungsgemäßen UV-Licht absorbierenden quartären Polysiloxans der allgemeinen Formel:

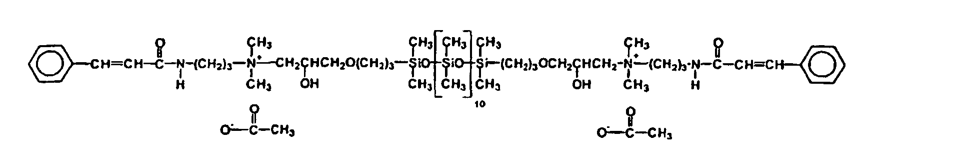

In einem 1 l-Vierhalskolben, versehen mit Rührer, Tropftrichter, Thermometer und Rückflusskühler, werden 139,6 g (0,6 Mol) eines tertiären Amins der allgemeinen Formel: 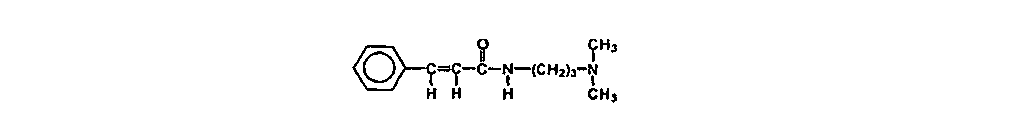 zusammen mit 36,3 g (0,6 Mol) Essigsäure und 120 ml Isopropanol vorgelegt. Nach 30 Minuten werden 330 g (0,3 Mol) eines Epoxysiloxans der allgemeinen Formel: 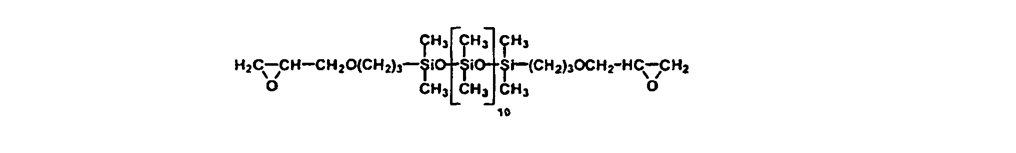 zugetropft, auf Rückflusstemperatur erwärmt und 6 Stunden gerührt. Anschließend wird bei 100 °C im Vakuum destilliert. Es wird ein hochviskoses, gelb-braunes Produkt erhalten (Quarternärer Stickstoff gef.: 1,4 %; theor.: 1,7 %.).

### UV-vis Spektrum Beispiel 1:

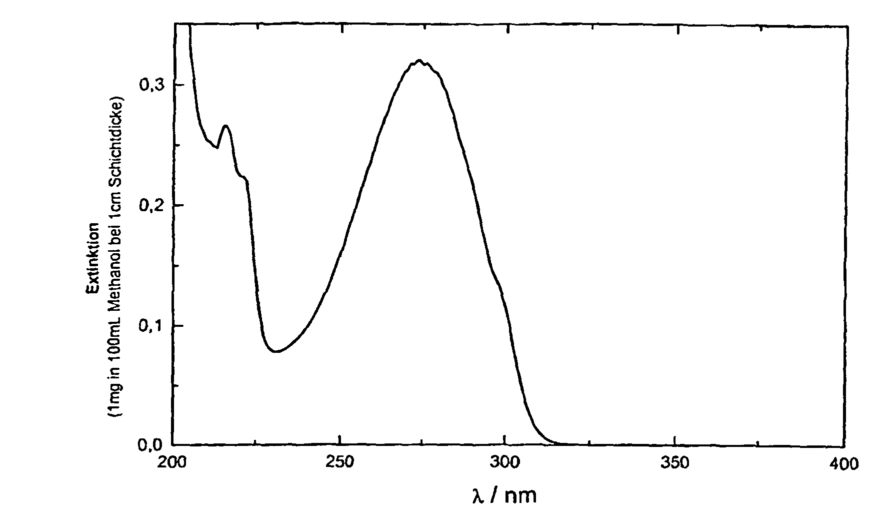

### Beispiel 2:

Herstellung eines erfindungsgemäßen UV-Licht absorbierenden quartären Polysiloxans der allgemeinen Formel:

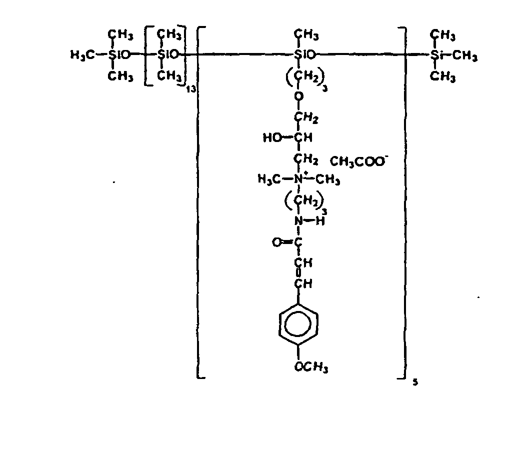

In einem 1 1-Vierhalskolben, versehen mit Rührer, Tropftrichter, Thermometer und Rückflusskühler, werden 158,0 g (0,6 Mol) eines tertiären Amins der allgemeinen Formel: 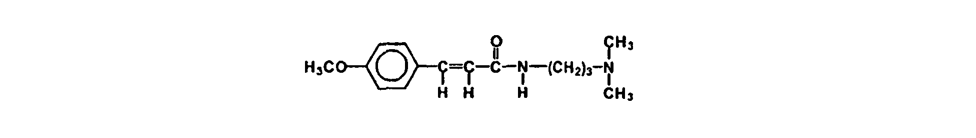 zusammen mit 36,3 g (0,6 Mol) Essigsäure und 120 ml Isopropanol vorgelegt. Nach 30 Minuten werden 240 g (0,12 Mol) eines Epoxysiloxans der allgemeinen Formel: 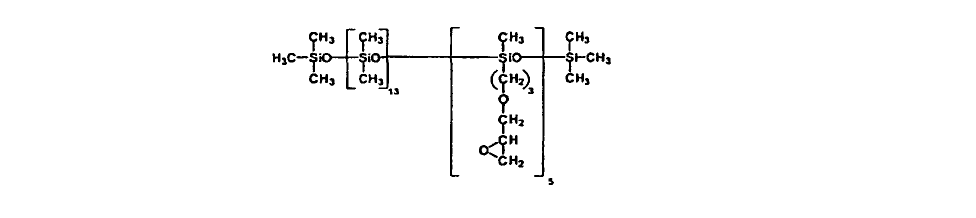 zugetropft, auf Rückflusstemperatur erwärmt und 6 Stunden gerührt. Anschließend wird bei 100 °C im Vakuum destilliert. Es wird ein hochviskoses, gelb-braunes Produkt erhalten (Quarternärer Stickstoff gef.: 1,65 %; theor.: 1,9 %.).

### UV-vis Spektrum Beispiel 2 :

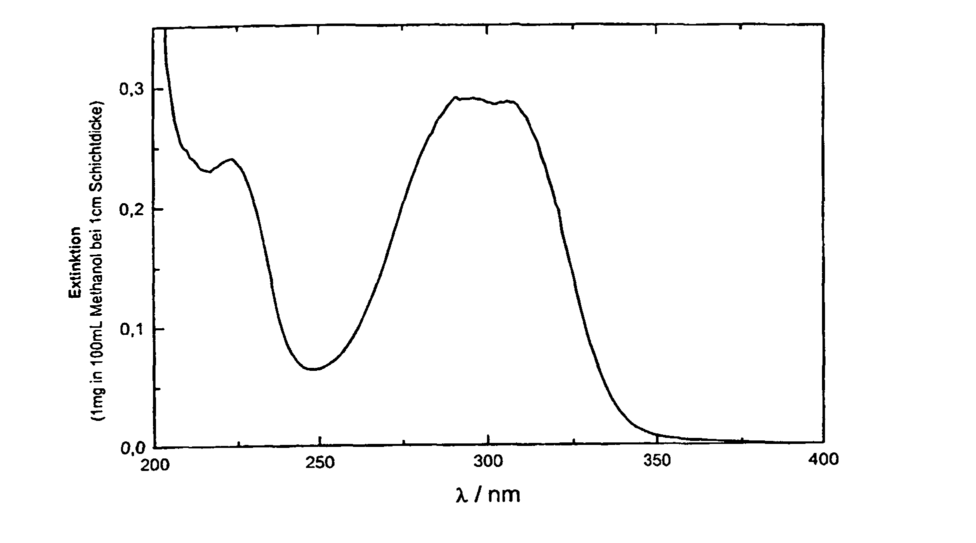

### Beispiel 3:

Herstellung eines erfindungsgemäßen UV-Licht absorbierenden quartären Polysiloxans der allgemeinen Formel:

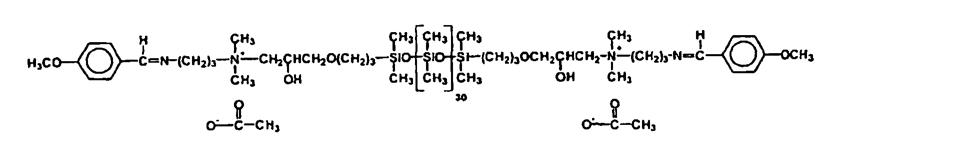

In einem 1,5 1-Vierhalskolben, versehen mit Rührer, Tropftrichter, Thermometer und Rückflusskühler, werden 132,0 g (0,6 Mol) eines tertiären Amins der allgemeinen Formel: 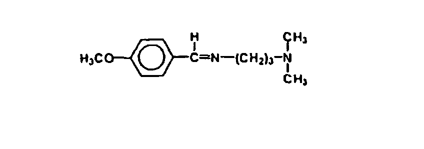 zusammen mit 36,3 g (0,6 Mol) Essigsäure und 120 ml Isopropanol vorgelegt. Nach 30 Minuten werden 774 g (0,3 Mol) eines Epoxysiloxans der allgemeinen Formel: 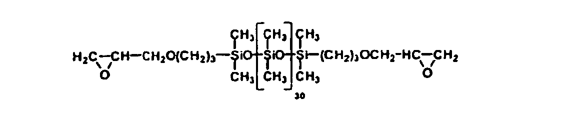 zugetropft, auf Rückflusstemperatur erwärmt und 6 Stunden gerührt. Anschließend wird bei 100 °C im Vakuum destilliert. Es wird ein hochviskoses, gelb-braunes Produkt erhalten (Quarternärer Stickstoff gef.: 0,7 %; theor.: 0,9 %.).

### UV-vis Spektrum Beispiel 3 :

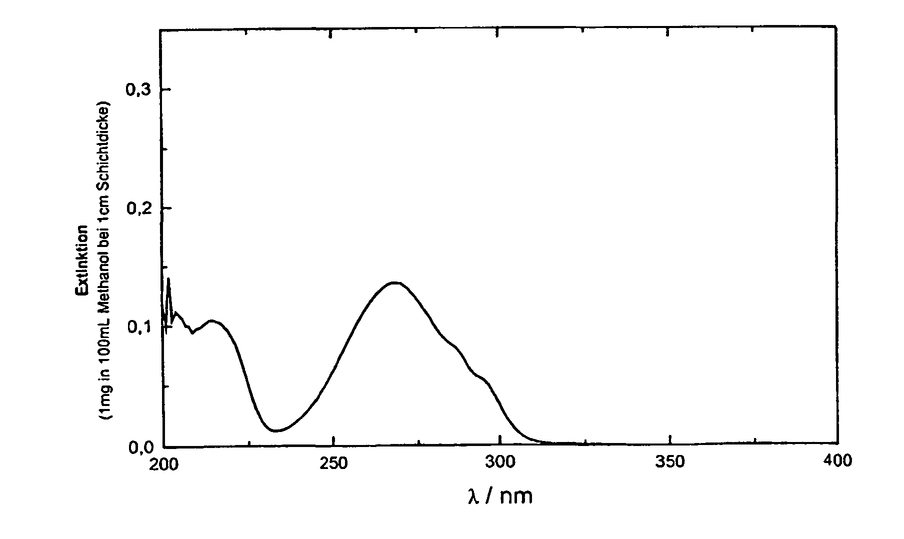

### Anwendungstechnischer Vergleich:

Für den anwendungstechnischen Vergleich wurden folgende erfindungsgemäße UV-Licht absorbierende quartäre Polysiloxane eingesetzt:

### Verbindung 1:

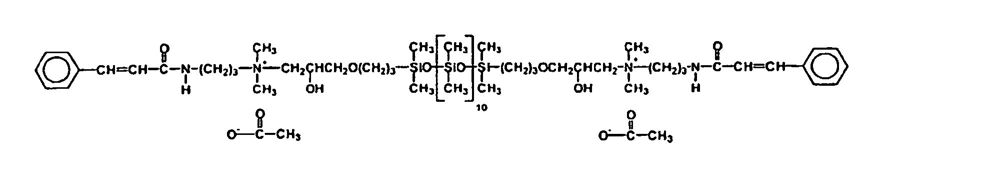

### Verbindung 2:

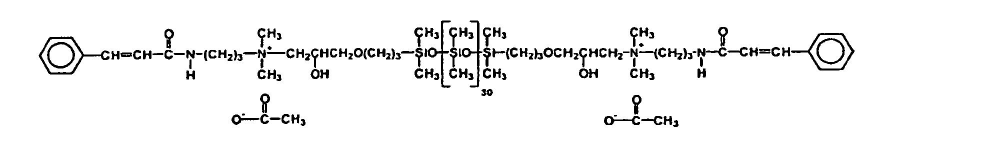

### Verbindung 3:

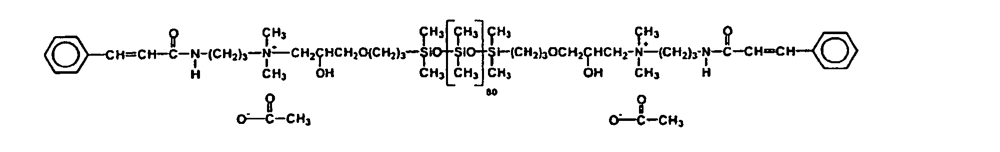

### Verbindung 4:

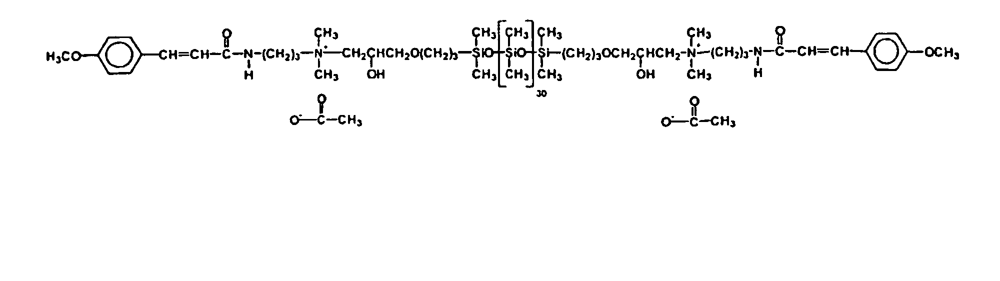

### Verbindung 5:

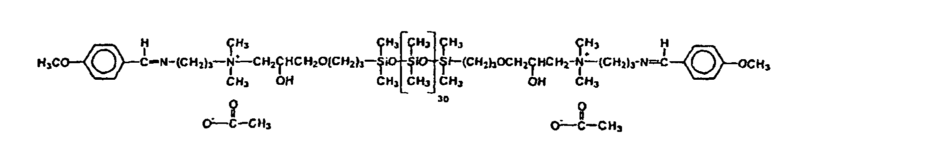

### Verbindung 6:

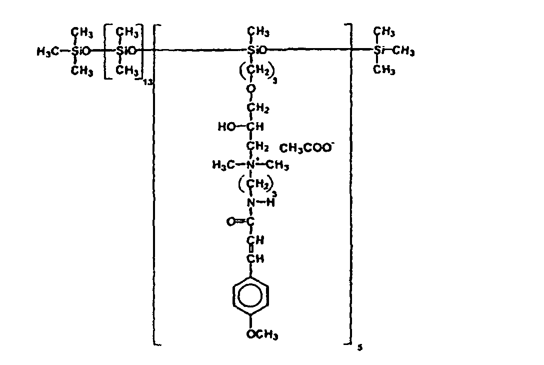

### Verbindung 7:

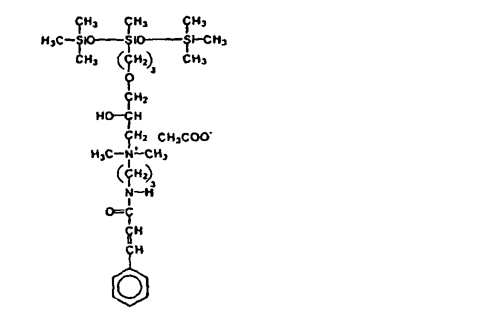

Herstellung und Überprüfung von Haarbehandlungsmitteln unter Verwendung der erfindungsgemäßen Verbindungen 1 bis 6:

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet.

### Materialien:

- Dauerwell-Flüssigkeit (z.B. "ondi", Wella)
- Fixierung (z.B. "neutrafix", Wella)
- Blondierpulver (z.B. "blondor special", Wella)
- H₂O₂ (e.g. "Welloxyd 9 %", Wella)
- Shampoo ohne Pflegeanteil (z.B. Natriumlaurylethersulfat (12 % WAS), NaCl verdickt)
- Bechergläser
- Haar-Färbe-Pinsel

Die Behandlung wird in der folgenden Reihenfolge durchgeführt:
1. Dauerwell-Behandlung:
   Die Haartressen werden mit der Dauerwellflüssigkeit angefeuchtet (Gewichtsverhältnis Haar : Flüssigkeit = 1 : 2). Nach einer Einwirkzeit von 15 min bei Raumtemperatur in einem abgedeckten Becherglas wird die Dauerwellflüssigkeit 2 min sorgfältig ausgespült. Anschließend werden die Haarsträhnchen mit einem Handtuch sanft ausgedrückt.
   Die Fixierung (Verhältnis Haar : Flüssigkeit = 1 : 2) hat eine Einwirkzeit von 10 min bei Raumtemperatur. Anschließend wird die Fixierung sorgfältig 2 min ausgespült.
   Die Haare werden anschließend über Nacht bei Raumtemperatur getrocknet.
2. Bleich-Behandlung:

Das Blondierpulver und das H₂O₂ werden zu einer Paste (Gewichtsverhältnis Pulver : H₂O₂ = 2 : 3) verarbeitet. Dann wird die Paste sofort auf die dauergewellten Haare mit einem Pinsel aufgetragen. Die Einwirkzeit beträgt 30 min bei Zimmertemperatur. Anschließend wird die Blondierpaste unter fließendem Wasser 2 min ausgespült.

Dann wird das Haar mit einem Shampoo ohne Conditioner 1 min gewaschen (Shampoomenge: 0,5 ml/Hairtress) und dann für 1 min ausgespült.

Bevor die vorgeschädigten Haarsträhnchen für Sensorik-Tests verwendet werden, werden sie über Nacht bei Raumtemperatur getrocknet.

### Testrezeptur:

Die konditionierenden Produkte werden in einer einfachen Haarspülung mit folgendem Aufbau

| Produkt | Gewichtsanteile |
|---|---|
| TEGINACID®C Ceteareth-25 | 0,5 % |
| TEGO®Alkanol 16 Cetyl Alcohol | 2,0 % |
| "Conditioner" | 2,0 % |
| Wasser | ad. 100 % |
| Zitronensäure | ad. pH 4,0 ± 0,3 |

getestet.

Als "Conditioner" sind die erfindungsgemäßen Verbindungsbeispiele bezeichnet.

Standardisierte Behandlung von vorgeschädigten Haarsträhnen mit konditionierenden Proben:

Die, wie oben beschrieben, vorgeschädigten Haarsträhnchen werden wie folgt mit der oben beschriebenen konditionierenden Spülung behandelt:

Die Haarsträhnen werden unter fließendem, warmem Wasser benetzt. Das überschüssige Wasser wird leicht von Hand ausgedrückt, dann wird die Spülung aufgebracht und sanft im Haar eingearbeitet (1 ml/Haarsträhne (2 g)). Nach einer Verweilzeit von 1 min wird das Haar für 1 min gespült.

Vor der sensorischen Beurteilung wird das Haar an der Luft bei 50 % Luftfeuchtigkeit und 25 °C für mindestens 12 h getrocknet.

### Beurteilungskriterien:

Die sensorischen Bewertungen erfolgt nach Noten, die auf einer Skala von 1 bis 5 vergeben werden, wobei 1 die schlechteste und 5 die beste Bewertung ist.

### Nasskämmbarkeit:

| Bewertung | Zahnung des Kamms | Ergebnis |
|---|---|---|
| 5 | grob | keine Knoten, das Haar ist leicht entwirrbar |
| | fein | sehr leichtes Durchkämmen, kein Widerstand spürbar |
| 4 | grob | einzelne Knoten, das Haar ist leicht entwirrbar |
| | fein | leichtes Durchkämmen, geringer Widerstand spürbar |
| 3 | grob | wenige Knoten, geringer Widerstand |
| | fein | etwas Widerstand spürbar, der nach mehrmaligem Kämmen nachlässt |
| 2 | grob | einige Knoten, merklicher Widerstand. |
| | fein | merklicher Widerstand, der nach mehrmaligem Kämmen nicht nachlässt. |
| 1 | grob | viele Knoten, starker Widerstand |
| | fein | sehr starker Widerstand, manchmal ist das Haar nicht durchkämmbar |

### Nassgriff:

| Bewertung | Ergebnis |
|---|---|
| 5 | sehr glatt, weich aber trotzdem schön fest, griffig, nicht schmierig/klebrig (keine Rückstände fühlbar) |
| 4 | glatt und weich und/oder nur geringe Rückstände spürbar |
| 3 | glatt, etwas hart und/oder etwas Rückstände spürbar |
| 2 | hart und/oder merkliche schmierige, wachsige Rückstände |
| 1 | sehr hart, rauh, stumpf und/oder extrem schmierig, klebrig (deutlich spürbare schmierige, wachsige Rückstände spürbar) |

### Trockenkämmbarkeit:

| Bewertung | Zahnung des Kamms | Ergebnis |
|---|---|---|
| 5 | grob | keine Knoten, das Haar ist leicht entwirrbar |
| | fein | sehr leichtes Durchkämmen, kein Widerstand spürbar, das Haar lädt sich nicht auf |
| 4 | grob | einzelne Knoten, das Haar ist leicht entwirrbar |
| | fein | leichtes Durchkämmen, geringer Widerstand sprürbar, Haar lädt sich minimal auf |
| 3 | grob | Wenige Knoten, geringer Widerstand |
| | fein | etwas Widerstand spürbar, der nach mehrmaligem Kämmen nachlässt, Haar lädt sich wenig auf |
| 2 | grob | einige Knoten, merklicher Widerstand |
| | fein | Merklicher Widerstand, der nach mehrmaligem Kämmen nicht nachlässt, Haar lädt sich auf |
| 1 | grob | viele Knoten, starker Widerstand |
| | fein | sehr starker Widerstand, manchmal ist das Haar nicht durchkämmbar, Haar lädt sich deutlich auf |

### Trockengriff:

| Bewertung | Ergebnis |
|---|---|
| 5 | sehr glatt, weich aber trotzdem fest, voll, griffig |
| 4 | glatt und weich |
| 3 | glatt, leicht hart und/oder leicht stumpf (Rückstände) |
| 2 | hart, etwas stumpf |
| 1 | rauh, hart, trocken, stumpf (Rückstände) |

### Trocken-Aussehen:

| Bewertung | Ergebnis |
|---|---|
| 5 | extrem glänzend |
| 4 | glänzend |
| 3 | etwas glänzend |
| 2 | wenig glänzend, leicht stumpf |
| 1 | stumpf, kein Glanz |

### Volumen:

Um das Volumen zu beurteilen, wird die Haarsträhne leicht geschüttelt, indem sie an der Klebestelle gehalten wird.

| Bewertung | Ergebnis |
|---|---|
| 5 | lockerer, bauschiger Fall, ⌀ im Spitzenbereich rel. groß |
| 4 - 2 | Zwischenstufen |
| 1 | Haar hängt schwer herab, ⌀ unterhalb der Bündelung ähnlich dem Spitzenbereich |

In der folgenden Tabelle 1 werden die Ergebnisse der sensorischen Beurteilung der wie oben beschrieben durchgeführten Behandlung der Haarstränchen mit erfindungsgemäßen Substanzen bzw. Placebo verglichen.

**Tabelle 1:**

| Erfindungsgemäße Beispiele | Detangling | Naßkämmbarkeit | Naßgriff | Trockenkämmbarkeit | Trockengriff | Glanz | Volumen |
|---|---|---|---|---|---|---|---|
| Verbindung 3 | 4,5 | 5 | 4 | 4,5 | 4,0 | 3,75 | 1,75 |
| Verbindung 4 | 4,5 | 4,5 | 3,75 | 4 | 4,25 | 3,75 | 1,5 |
| Verbindung 2 | 3,5 | 4 | 3,75 | 4 | 3,75 | 3,5 | 1,75 |
| Verbindung 5 | 4 | 3,75 | 4,25 | 3,75 | 3,75 | 3,75 | 1,5 |
| Verbindung 1 | 3,5 | 3,5 | 4 | 3,5 | 3,5 | 3,5 | 2 |
| Verbindung 6 | 3,5 | 3,25 | 3,5 | 3,5 | 3,25 | 3,5 | 2 |
| Placebo | 1,5 | 1,25 | 1,75 | 2,25 | 2,75 | 3,25 | 2,5 |

Es zeigt sich, dass die erfindungsgemäßen Verbindungsbeispiele in der sensorischen Beurteilung sehr gute kosmetische Bewertungen erhalten.

Überprüfung von Textilweichspülern unter Verwendung der erfindungsgemäßen Verbindungen 1 bis 7:

Zur Überprüfung der Weichheit und der UV-Schutzwirkung auf textilen Geweben werden aus marktüblichen Esterquats (z.B. Rewoquat® WE 18) 18 %ige Weichspülformulierungen hergestellt, die jeweils zwischen 1 % und 5 % Zusätze der Verbindungen 1 bis 6 enthalten. Die Weichheit wird an handelsüblichem Baumwollfrottiergewebe und die UV-Schutzwirkung an gefärbten Testgeweben gemäß USTM-Standards gemessen. Als Testfarben werden Direct Blue 1 (DB 1), Direkt Blue 90 (DB 90) und Acid Red 151 (AR 151) eingesetzt.

Die Formulierungen mit den Zusätzen der Verbindungen 1 bis 6 sollten einen positiven Effekt auf die Weichheit der Gewebe aufweisen, wobei auch ein neutrales Verhalten akzeptabel ist. Ein negativer Effekt auf die Weichheit, führt zu einer Abwertung in der Gesamtbeurteilung.

Die Einsatzkonzentration aller Weichspülerformulierungen beträgt analog einer normalen Haushaltswaschmaschine 0,025 %.

Nach der Applikation werden die Frottiergewebe von einem Panel bestehend aus 10 geschulten Personen hinsichtlich ihres Weichgriffs beurteilt.

Alle silikonhaltigen Formulierungen werden direkt gegen das Wäscheweich ohne UV-Licht absorbierende quarternäre Polysiloxane (nur Rewoquat® WE 18) verglichen. Dabei bedeutet (1) = sehr weich und (5) = hart. Die Ergebnisse sind in Tabelle 2 zusammengefaßt.

Die gefärbten Testgewebe wurden 4 Wochen mit einer 1000 Watt Lampe bestrahlt, wobei deren Spektrum der des natürlichen Sonnenlichtes weitestgehend entspricht.

Vor und nach der Bestrahlung werden mittels eines Farbmessgerätes die Farbwerte (E) der gefärbten Gewebeläppchen gemessen. In Tabelle 2 sind jeweils die Differenzen der Farbwerte (ΔE) vor und nach der Bestrahlung angegeben.

**Tabelle 2:**

| Verbindung | Gehalt Polysiloxan | Weichgriff | Farbveränderung ΔE | | |
|---|---|---|---|---|---|
| | | | DB 1 | DB 90 | AR 151 |
| WE 18 | / | 2,0 | 7,4 | 5,9 | 4,9 |
| 1 | 1 % | 2,2 | 1,4 | 1,3 | 1,4 |
| 1 | 3 % | 2,1 | 0,9 | 0,6 | 0,6 |
| 1 | 5 % | 1,9 | 0,7 | 0,5 | 0,4 |
| 2 | 1 % | 1,6 | 2,9 | 2,6 | 2,1 |
| 2 | 3 % | 1,5 | 2,1 | 2,0 | 1,5 |
| 2 | 5 % | 1,7 | 1,6 | 1,2 | 0,9 |
| 3 | 1 % | 1,4 | 4,7 | 4,3 | 4,1 |
| 3 | 3 % | 1,3 | 3,8 | 3,6 | 3,4 |
| 3 | 5 % | 1,5 | 3,0 | 2,8 | 2,1 |
| 4 | 1 % | 1,6 | 2,7 | 2,5 | 2,2 |
| 4 | 3 % | 1,4 | 2,0 | 1,8 | 1,4 |
| 4 | 5 % | 1,8 | 1,5 | 1,3 | 0,9 |
| 5 | 1 % | 1,4 | 4,5 | 4,4 | 3,8 |
| 5 | 3 % | 1,4 | 3,5 | 3,8 | 3,4 |
| 5 | 5 % | 1,6 | 2,8 | 3,0 | 2,0 |
| 6 | 1 % | 2,1 | 1,5 | 1,0 | 1,3 |
| 6 | 3 % | 2 | 1,0 | 0,7 | 0,7 |
| 6 | 5 % | 2 | 0,7 | 0,4 | 0,4 |
| 7 | 1 % | 2 | 1,3 | 1,2 | 1,4 |
| 7 | 3 % | 2,2 | 0,9 | 0,7 | 0,6 |
| 7 | 5 % | 2,2 | 0,7 | 0,4 | 0,5 |

Es zeigt sich, dass die erfindungsgemäßen Verbindungen den Weichgriff gegenüber dem Standard WE 18 verbessern und eine Kontrolle über die schädigende UV-Strahlung ausüben.

## Patentansprüche

1. UV-Licht absorbierende quartäre Polysiloxane der allgemeinen Formel (I) 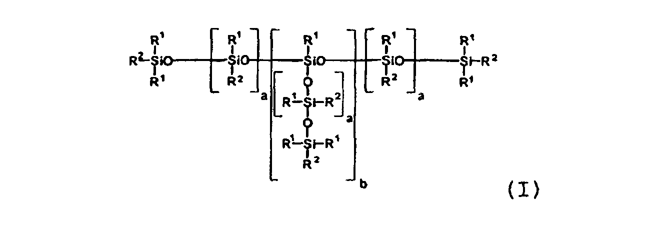 worin die Reste
R¹ gleich oder verschieden sind und jeweils niedere Alkylreste mit 1 bis 4 Kohlenstoffatomen oder Phenylreste bedeuten,
R² zu einem Teil die Bedeutung der Reste R¹ haben können und die übrigen Reste R² Reste der Formel (Ia)
R² = -M-Z⁺ A⁻ (Ia)
sind, mit der Maßgabe, dass im durchschnittlichen Molekül mindestens ein Rest R² ein Rest der Formel -M-Z⁺ A⁻ ist,
worin
Z ein Rest der Formel (Ib) 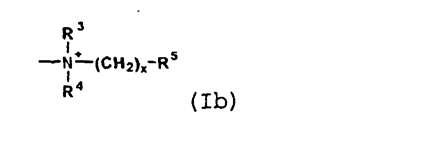 ist,
R³, R⁴ Alkylreste mit 1 bis 22 Kohlenstoffatomen oder Alkenylreste mit 2 bis 22 Kohlenstoffatomen, worin die Alkyl- oder Alkenylreste Hydroxylgruppen aufweisen können,
R⁵ ein einwertiger, für die UV-Absorption verantwortlicher Chromophorrest, der Formel (Ic) 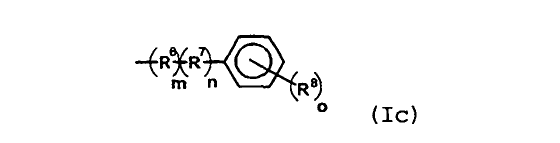 worin
R⁶ 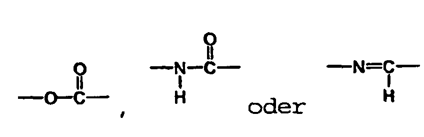 ist,
R⁷ -CH=CH- ist,
R⁸ gleich oder verschieden sind und jeweils Wasserstoff, Alkyl-, Haloalkyl-, Halogen-, Phenyl-, Hydroxyl-, Alkoxy-, Amino-, Alkylamino-, Dialkylamino-, Di(hydroxyalkyl)amino- oder Di (polyalkoxy)aminoreste bedeuten,
m = 0 oder 1,
n = 0 oder 1,
o = 0 bis 5,
x = 0 bis 6,
M ein zweiwertiger Kohlenwasserstoffrest mit mindestens 4 Kohlenstoffatomen ist, der eine Hydroxylgruppe aufweist und der durch ein oder mehrere Sauerstoffatome unterbrochen sein kann, wobei das N-Atom des Restes Z mit dem Rest M über das zur C-OH-Gruppe im Rest M benachbarte Kohlenstoffatom verbunden ist,
A⁻ ein anorganisches oder organisches Anion ist, das von einer üblichen physiologisch verträglichen Säure HA herrührt,
a unabhängig voneinander einen Wert von 1 bis 200 und
b einen Wert von 0 bis 10 hat.

2. UV-Licht absorbierende Verbindungen gemäß Anspruch 1, **dadurch gekennzeichnet, dass** R¹ ein Methyl-, Ethyl-, n-Propyl-, iso-Propyl-, n-Butylrest, iso-Butylrest, oder Phenylrest sein kann.

3. UV-Licht absorbierende Verbindungen gemäß den Ansprüchen 1 und 2, **dadurch gekennzeichnet, dass** M gleich oder verschieden einer der Reste ist, ausgesucht aus der Gruppe

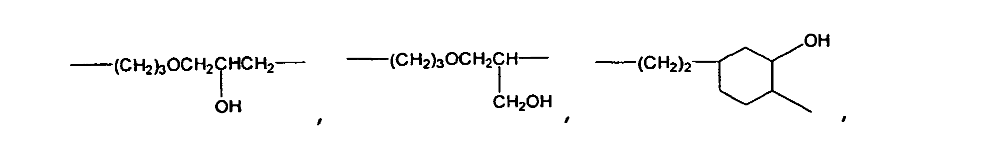

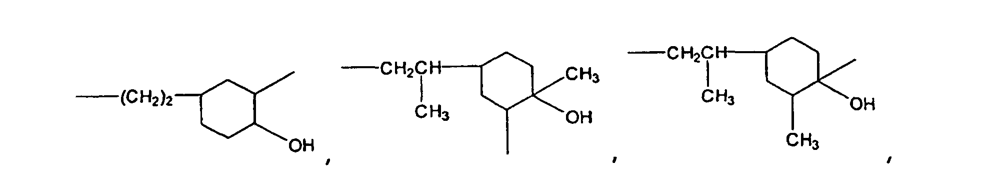

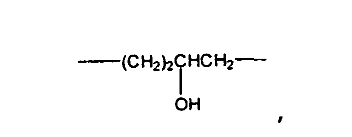

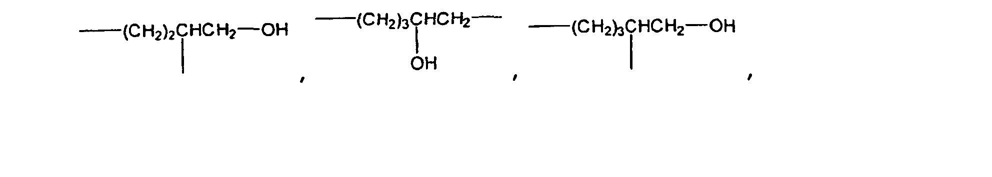

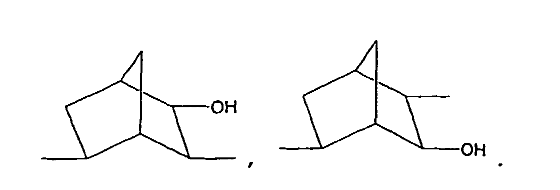

4. UV-Licht absorbierende Verbindungen gemäß den Ansprüchen 1 bis 3, **dadurch gekennzeichnet, dass** Z gleich oder verschieden einer der Reste ist, ausgesucht aus der Gruppe

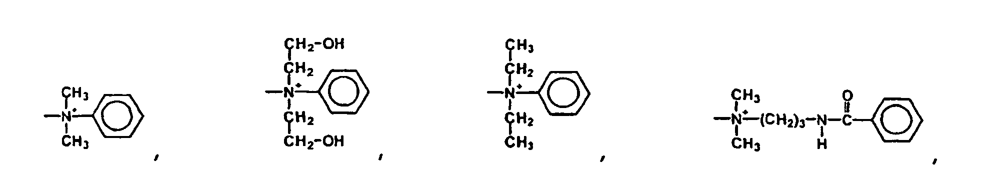

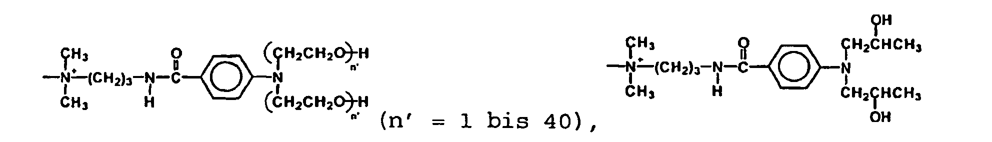

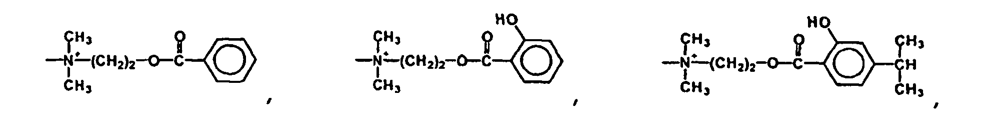

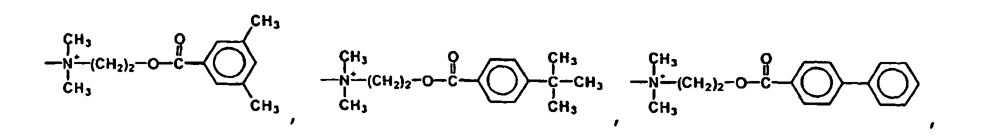

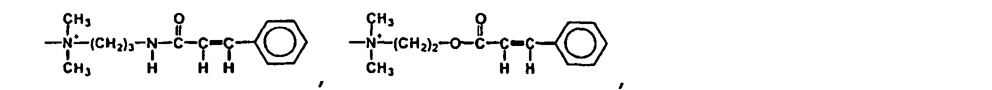

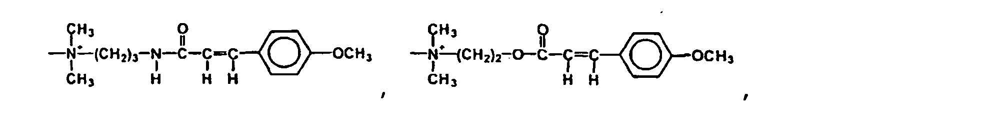

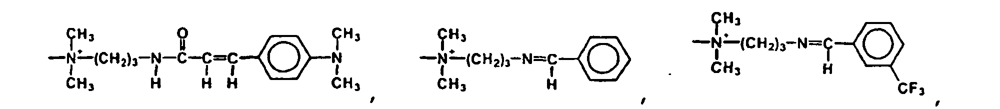

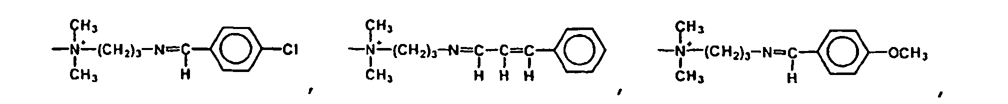

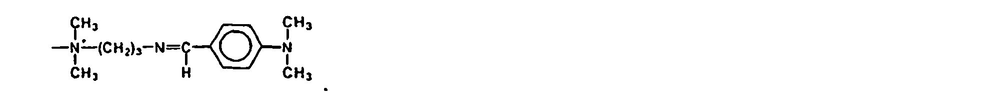

5. UV-Licht absorbierende Verbindungen gemäß den Ansprüchen 1 bis 4, **dadurch gekennzeichnet, dass** A⁻ gleich oder verschieden einer der Reste ist, ausgesucht aus der Gruppe Acetat-, Chlorid-, Bromid-, Hydrogensulfat-, Sulfat-, Methosulfat-, Ethosulfat-, Citrat-, Tartrat- und Lactationen, sowie Anionen aromatischer Säuren, wie die Anionen der p-Toluolsulfonsäure, Benzoesäure, Salicylsäure, Zimtsäure, 4-Methoxyzimtsäure, 4-Aminobenzoesäure, 4-Bis-(hydroxypropyl)aminobenzoesäure, 4-Bis(polyethoxy)aminobenzoesäure, 4-Dimethylaminobenzoesäure, 3-Imidazol-4-yl-acrylsäure, 2-Phenylbenzimidazol-5-sulfonsäure, 3,3'-(1,4-Phenylendimethin)-bis(7,7-dimethyl-2-oxo-bicyclo[2.2.1]heptan-1-methansulfonsäure), 2-Hydroxy-4-methoxybenzophenon-5-sulfonsäure und 3-(4'-Sulfo)benzyliden-bornan-2-on.

6. UV-Licht absorbierende Verbindung der Formel

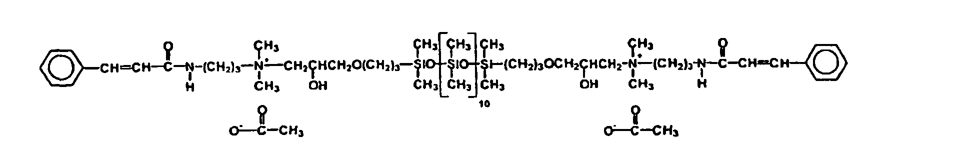

7. UV-Licht absorbierende Verbindung der Formel

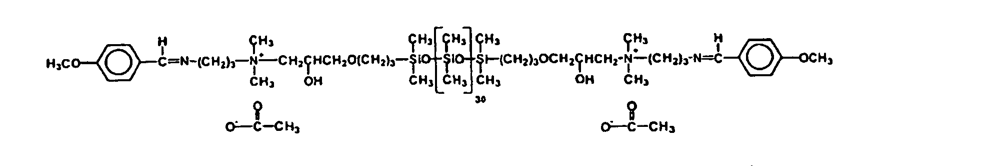

8. UV-Licht absorbierende Verbindung der Formel

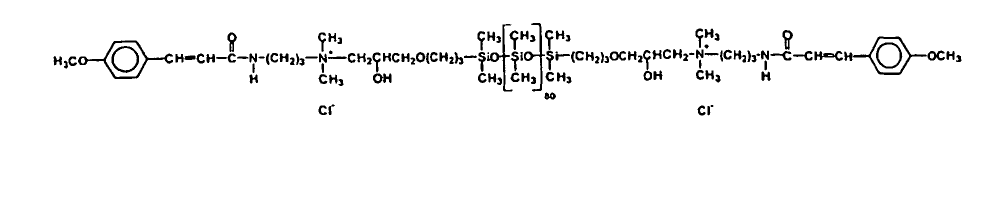

9. UV-Licht absorbierende Verbindung der Formel

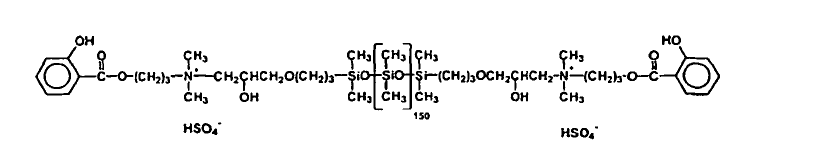

10. UV-Licht absorbierende Verbindung der Formel

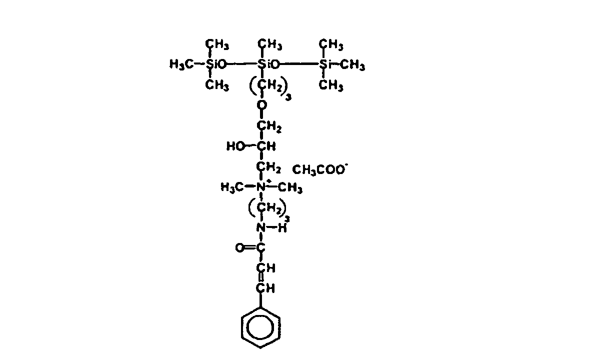

11. UV-Licht absorbierende Verbindung der Formel

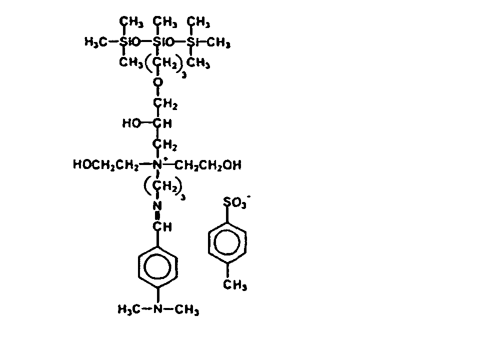

12. UV-Licht absorbierende Verbindung der Formel

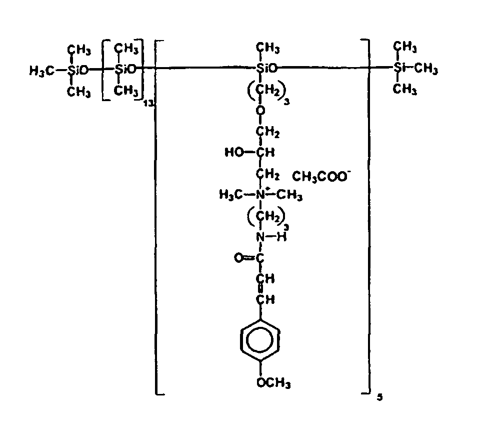

13. Verwendung der Verbindungen der allgemeinen Formel (I) zur Herstellung von UV-Licht absorbierenden Formulierungen.

14. Verwendung der Verbindungen der allgemeinen Formel (I) zur Herstellung von UV-Licht absorbierenden kosmetischen Formulierungen.

15. Verwendung der Verbindungen der allgemeinen Formel (I) zur Herstellung von UV-Licht absorbierenden Wäscheweichspülmitteln.

16. Verwendung der Verbindungen der allgemeinen Formel (I) zur Herstellung von UV-Licht absorbierenden Haarreinigungs- und pflegemitteln.

17. Verwendung der Verbindungen der allgemeinen Formel (I) zur Herstellung von UV-Licht absorbierenden Hautschutz-, Hautreinigungs- und/oder Hautpflegemitteln.

18. Verfahren zur Herstellung von UV-Licht absorbierenden Verbindungen, **dadurch gekennzeichnet, dass** man Verbindungen der allgemeinen Formel (II) 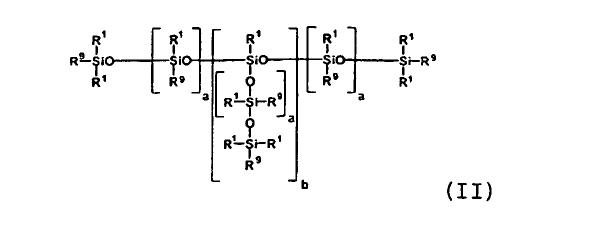 worin die Reste
R¹ gleich oder verschieden sind und jeweils niedere Alkylreste mit 1 bis 4 Kohlenstoffatomen oder Phenylreste bedeuten,
R⁹ zu einem Teil die Bedeutung der Reste R¹ haben können und die übrigen Reste R⁹ einwertige Reste sind, welche der Struktur des Restes M entsprechen, wobei der Rest R⁹ anstelle der Verknüpfung mit Z und der Hydroxylgruppe eine Epoxidgruppe aufweist,
mit der Maßgabe, dass im durchschnittlichen Molekül mindestens ein Rest R⁹ ein einwertiger Rest ist, welcher der Struktur des Restes M entspricht, wobei der Rest R⁹ anstelle der Verknüpfung mit Z und der Hydroxylgruppe eine Epoxidgruppe aufweist,
a unabhängig voneinander einen Wert von 1 bis 200 und
b einen Wert von 0 bis 10 hat,
mit tertiären Aminen der allgemeinen Formel (IIb) 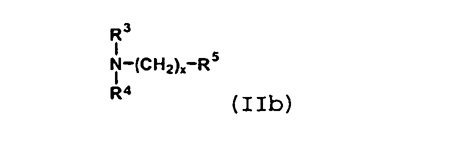 worin
R³, R⁴ Alkylreste mit 1 bis 22 Kohlenstoffatomen oder Alkenylreste mit 2 bis 22 Kohlenstoffatomen, worin die Alkyl- oder Alkenylreste Hydroxylgruppen aufweisen können,
R⁵ ein einwertiger, für die UV-Absorption verantwortlicher Chromophorrest, der Formel (Ic) 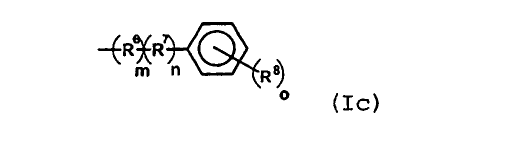 worin
R⁶ 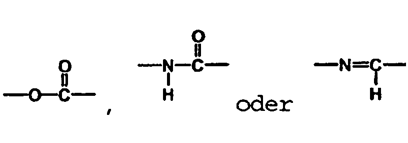 ist,
R⁷ -CH=CH- ist,
R⁸ gleich oder verschieden sind und jeweils Wasserstoff, Alkyl-, Haloalkyl-, Halogen-, Phenyl-, Hydroxyl-, Alkoxy-, Amino-, Alkylamino-, Dialkylamino-, Di(hydroxyalkyl)amino- oder Di (polyalkoxy) aminoreste bedeuten,
m = 0 oder 1,
n = x0 oder 1,
o = 0 bis 5,
x = 0 bis 6,
in an sich bekannter Weise in solchen Mengenverhältnissen umsetzt, dass jeder Epoxidgruppe mindestens eine tertiäre Aminogruppe entspricht, und man die Umsetzung in Gegenwart eines üblichen physiologisch verträglichen organischen oder anorganischen Säureequivalentes HA, bezogen auf zu quaternisierendes Stickstoffatom, und bei Temperaturen von 40 bis 120 °C durchführt.

## Claims

1. UV-light-absorbing quaternary polysiloxanes of the general formula (I) 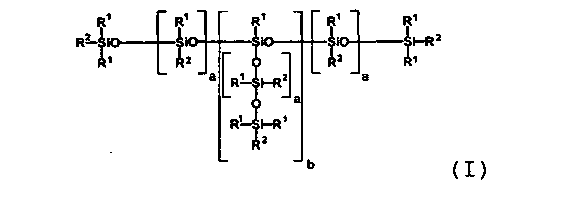 in which the radicals
R¹ are identical or different and are in each case lower alkyl radicals having 1 to 4 carbon atoms or phenyl radicals,
R² may in part have the meaning of the radicals R¹, and the other radicals R² are radicals of the formula (Ia)
R² = -M-Z⁺ A⁻ (Ia)
with the proviso that, in the average molecule, at least one radical R² is a radical of the formula -M-Z⁺ A⁻,
in which
Z is a radical of the formula (Ib) 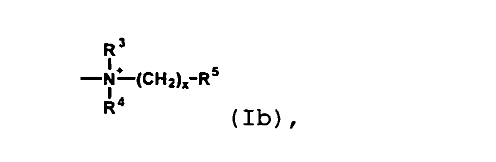
R³, R⁴ are alkyl radicals having 1 to 22 carbon atoms or alkenyl radicals having 2 to 22 carbon atoms, in which the alkyl or alkenyl radicals may have hydroxyl groups,
R⁵ is a monovalent chromophore radical responsible for UV-absorption of the formula (Ic) 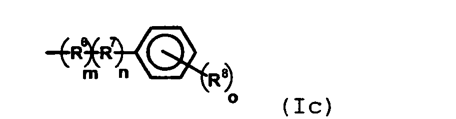 in which
R⁶ is 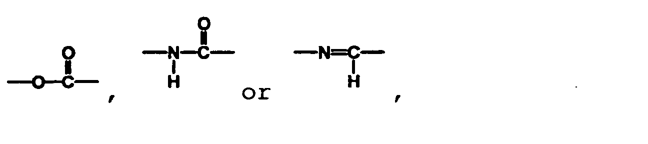
R⁷ is -CH=CH-,
R⁸ are identical or different and in each case are hydrogen, alkyl, haloalkyl, halogen, phenyl, hydroxyl, alkoxy, amino, alkylamino, dialkylamino, di(hydroxyalkyl)amino or di(polyalkoxy)amino radicals,
m = 0 or 1,
n = 0 or 1,
o = 0 to 5,
x = 0 to 6,
M is a divalent hydrocarbon radical having at least 4 carbon atoms which has one hydroxyl group and which may be interrupted by one or more oxygen atoms, where the N atom of the radical Z is bonded to the radical M via the carbon atom adjacent to the C-OH group in the radical M,
A⁻ is an inorganic or organic anion which stems from a customary physiologically compatible acid HA,
a, independently of the others, has a value from 1 to 200 and
b has a value from 0 to 10.

2. UV-light-absorbing compounds according to Claim 1, **characterized in that** R¹ may be a methyl, ethyl, n-propyl, isopropyl, n-butyl radical, isobutyl radical or phenyl radical.

3. UV-light-absorbing compounds according to Claims 1 and 2, **characterized in that** M is identical or different to one of the radicals chosen from the group

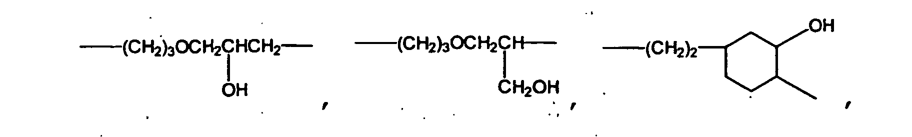

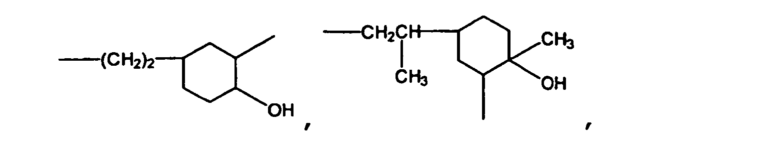

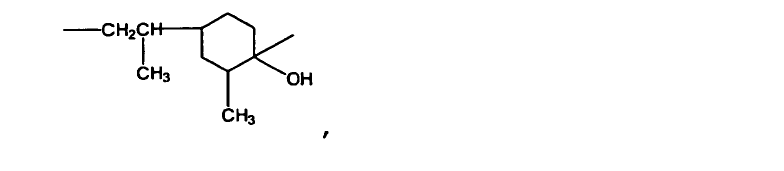

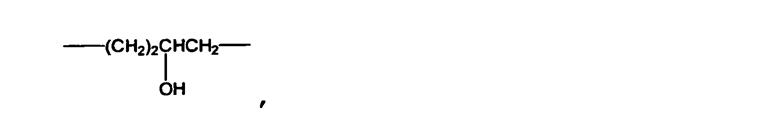

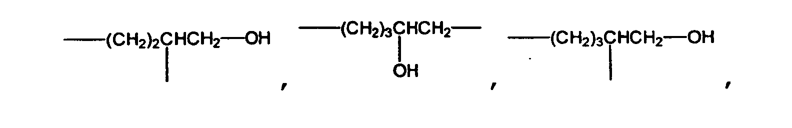

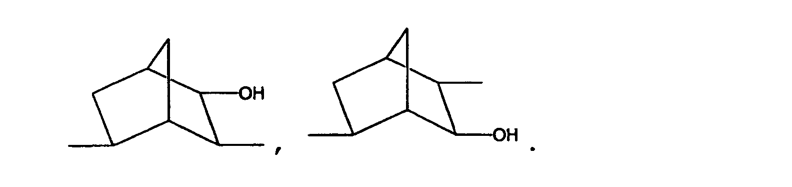

4. UV-light-absorbing compounds according to Claims 1 to 3, **characterized in that** Z is identical or different to one of the radicals chosen from the group

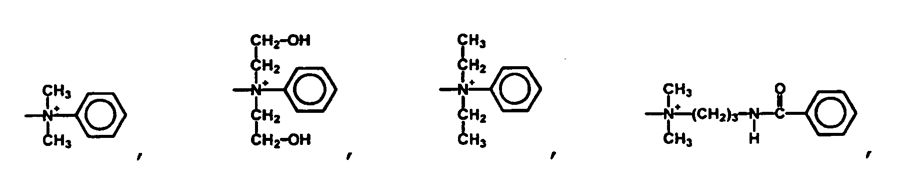

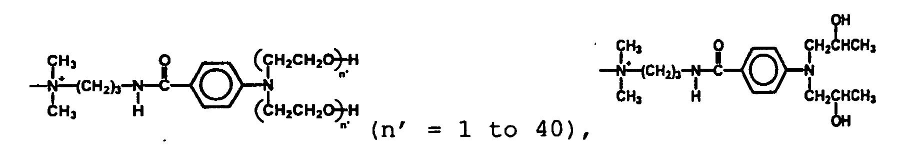

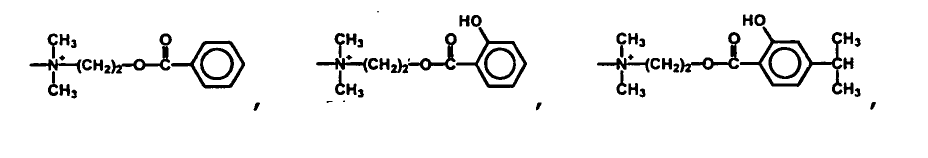

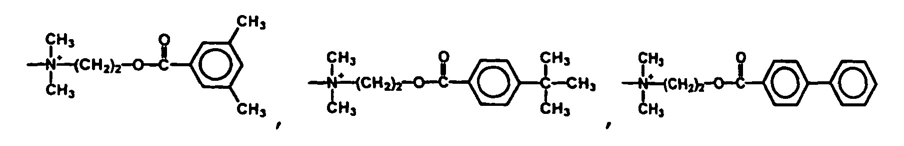

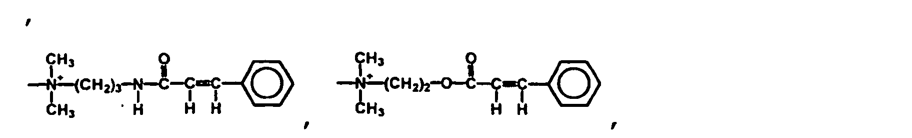

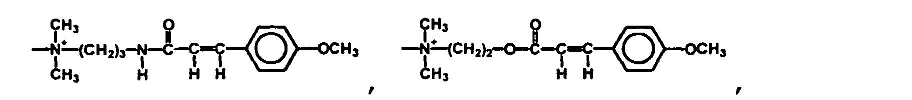

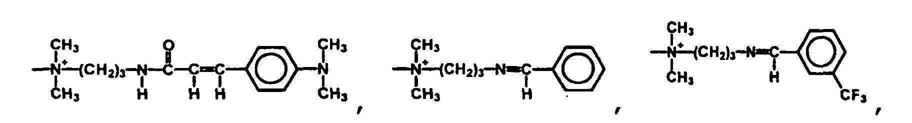

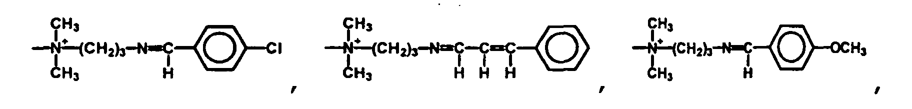

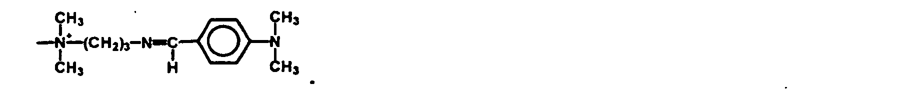

5. UV-light-absorbing compounds according to Claims 1 to 4, **characterized in that** A⁻ is identical or different to one of the radicals chosen from the group consisting of acetate, chloride, bromide, hydrogensulphate, sulphate, methosulphate, ethosulphate, citrate, tartrate and lactate ions, and anions of aromatic acids, such as the anions of p-toluenesulphonic acid, benzoic acid, salicylic acid, cinnamic acid, 4-methoxycinnamic acid, 4-aminobenzoic acid, 4-bis-(hydroxypropyl)aminobenzoic acid, 4-bis(polyethoxy)aminobenzoic acid, 4-dimethylaminobenzoic acid, 3-imidazol-4-ylacrylic acid, 2-phenylbenzimidazole-5-sulphonic acid, 3,3'-(1,4-phenylenedimethine)bis(7,7-dimethyl-2-oxo-bicyclo[2.2.1]heptane-1-methanesulphonic acid), 2-hydroxy-4-methoxybenzophenone-5-sulphonic acid and 3-(4'-sulpho)benzylidenebornan-2-one.

6. UV-light-absorbing compound of the formula

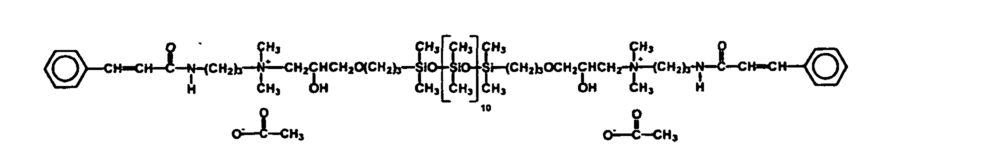

7. UV-light-absorbing compound of the formula

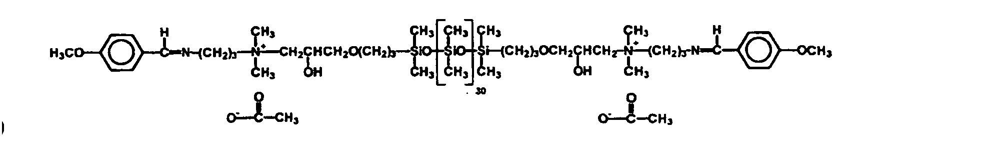

8. UV-light-absorbing compound of the formula

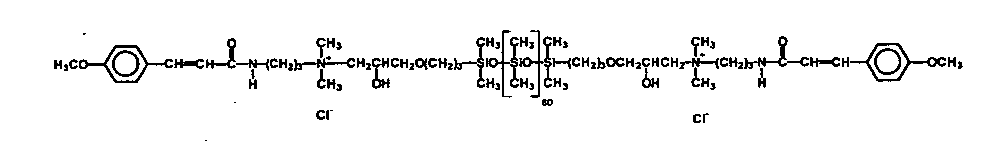

9. UV-light-absorbing compound of the formula

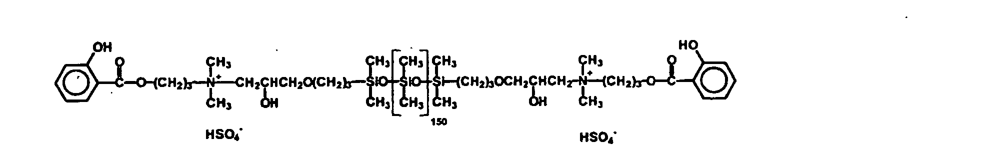

10. UV-light-absorbing compound of the formula

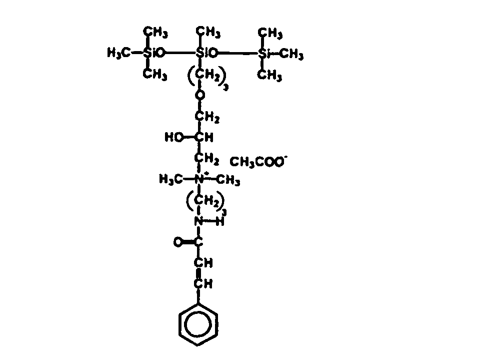

11. UV-light-absorbing compound of the formula

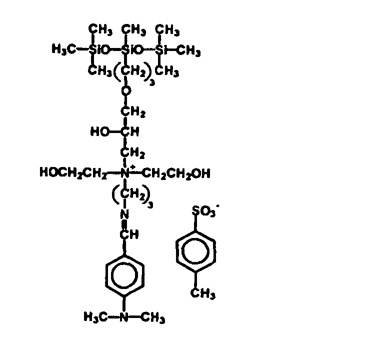

12. UV-light-absorbing compound of the formula

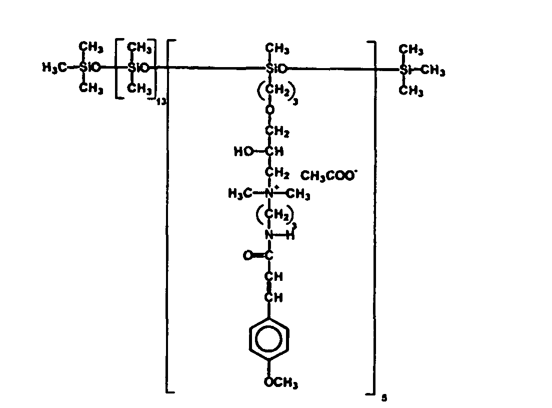

13. Use of the compounds of the general formula (I) for the preparation of UV-light-absorbing formulations.

14. Use of the compounds of the general formula (I) for the preparation of UV-light-absorbing cosmetic formulations.

15. Use of the compounds of the general formula (I) for the preparation of UV-light-absorbing fabric softeners.

16. Use of the compounds of the general formula (I) for the preparation of UV-light-absorbing hair cleansing and care compositions.

17. Use of the compounds of the general formula (I) for the preparation of UV-light-absorbing skin protection, skin cleansing and/or skin care compositions.

18. Process for the preparation of UV-light-absorbing compounds, **characterized in that** compounds of the general formula (II) are reacted 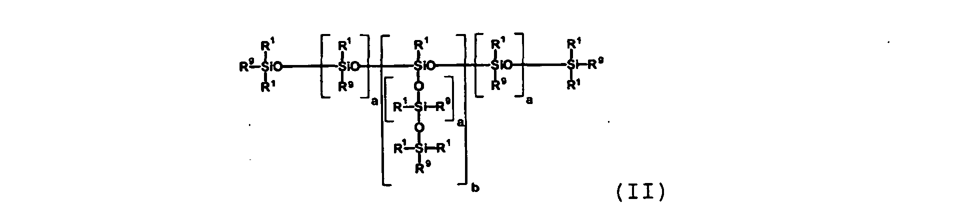 in which the radicals
R¹ are identical or different and in each case are lower alkyl radicals having 1 to 4 carbon atoms or phenyl radicals,
R⁹ may in part have the meaning of the radicals R¹, and the remaining radicals R⁹ are monovalent radicals which correspond to the structure of the radical M, where the radical R⁹ has an epoxide group in place of the linkage to Z and the hydroxyl group,
with the proviso that, in the average molecule, at least one radical R⁹ is a monovalent radical which corresponds to the structure of the radical M, where the radical R⁹ has an epoxide group in place of the linkage to Z and the hydroxyl group,
a independently of the others, has a value from 1 to 200 and
bhas a value from 0 to 10,
with tertiary amines of the general formula (IIb) 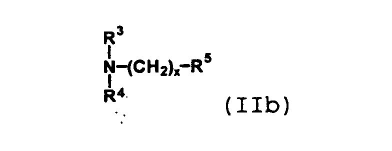 in which
R³, R⁴ are alkyl radicals having 1 to 22 carbon atoms or alkenyl radicals having 2 to 22 carbon atoms, in which the alkyl or alkenyl radicals may have hydroxyl groups,
R⁵ is a monovalent chromophore radical responsible for the UV-absorption of the formula (Ic) 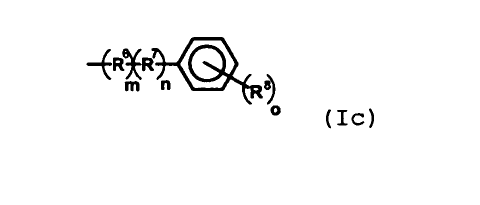 in which
R⁶ is 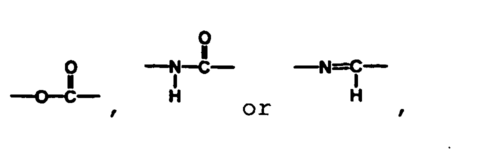
R⁷ is -CH=CH-,
R⁸ are identical or different and are in each case hydrogen, alkyl, haloalkyl, halogen, phenyl, hydroxyl, alkoxy, amino, alkylamino, dialkylamino, di(hydroxyalkyl)amino or di(polyalkoxy)amino radicals,
m = 0 or 1,
n = 0 or 1,
o = 0 to 5,
x = 0 to 6,
in a manner known per se in quantitative ratios such that each epoxide group corresponds to at least one tertiary amino group and the reaction is carried out in the presence of a customary physiologically compatible organic or inorganic acid equivalent HA, based on nitrogen atom to be quaternized, and at temperatures of from 40 to 120°C.

## Revendications

1. Polysiloxanes quaternaires absorbant la lumière UV de formule générale (I) 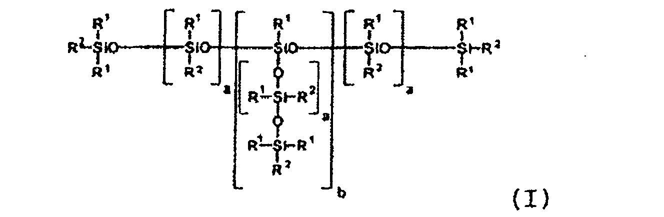 dans laquelle
les radicaux R¹ sont identiques ou différents et signifient à chaque fois des radicaux alkyle inférieurs, comprenant 1 à 4 atomes de carbone ou des radicaux phényle,
les radicaux R² peuvent présenter pour une partie la signification des radicaux R¹ et les autres radicaux R² sont des radicaux de formule (Ia)
R² = -M-Z⁺A⁻
à condition que dans la molécule moyenne, au moins un radical R² représente un radical de formule -M-Z⁺A⁻,
dans laquelle
Z représente un radical de formule (Ib) 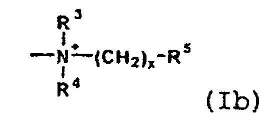 les radicaux R³, R⁴ représentent des radicaux alkyle comprenant 1 à 22 atomes de carbone ou des radicaux alcényle comprenant 2 à 22 atomes de carbone, où les radicaux alkyle ou alcényle peuvent présenter des groupements hydroxyle,
R⁵ représente un radical chromophore monovalent, responsable de l'absorption de la lumière UV, de formule (Ic) 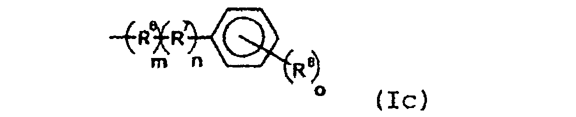 dans laquelle
R⁶ représente 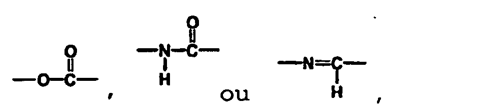 R⁷ représente -CH=CH-,
les radicaux R⁸ sont identiques ou différents et signifient à chaque fois hydrogène, des radicaux alkyle, halogénoalkyle, halogène, phényle, hydroxyle, alcoxy, amino, alkylamino, dialkylamino, di(hydroxyalkyl)amino ou di(polyalcoxy)amino,
m = 0 ou 1,
n = 0 ou 1,
o = 0 à 5,
x = 0 à 6,
M représente un radical hydrocarboné divalent comprenant au moins 4 atomes de carbone, qui présente un groupement hydroxyle et qui peut être interrompu par un ou plusieurs atomes d'oxygène, l'atome N du radical Z étant relié au radical M via l'atome de carbone adjacent au groupement C-OH dans le radical M,
A⁻ représente un anion inorganique ou organique, qui provient d'un acide HA usuel, physiologiquement acceptable,
a présente, indépendamment l'un de l'autre, une valeur de 1 à 200 et
b présente une valeur de 0 à 10.

2. Composés absorbant la lumière UV selon la revendication 1, **caractérisés en ce que** R¹ peut être un radical méthyle, éthyle, n-propyle, iso-propyle, n-butyle, iso-butyle ou phényle.

3. Composés absorbant la lumière UV selon les revendications 1 et 2, **caractérisés en ce que** M est identique ou différent et représente un des radicaux choisi dans le groupe constitué par les radicaux

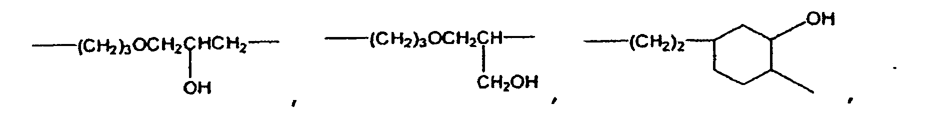

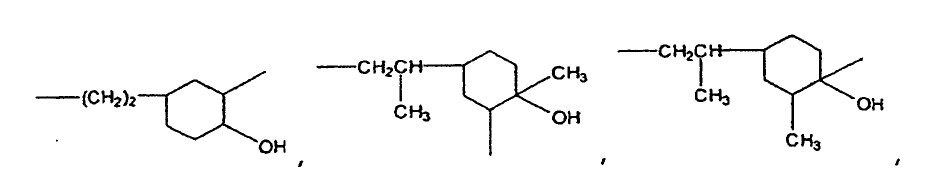

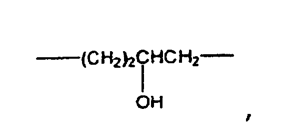

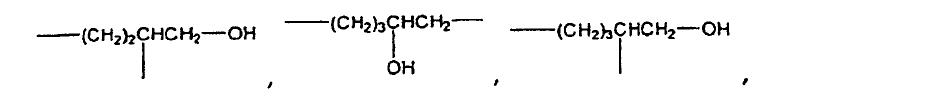

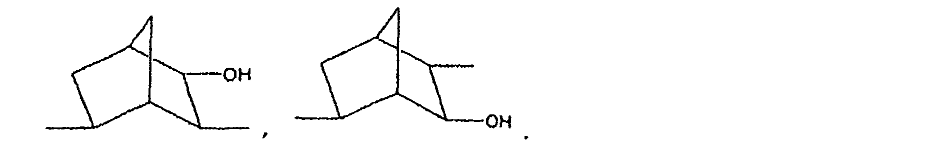

4. Composés absorbant la lumière UV selon les revendications 1 à 3, **caractérisés en ce que** Z est identique ou différent et représente un des radicaux choisi dans le groupe constitué par les radicaux

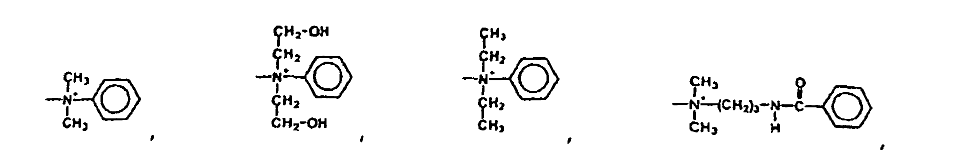

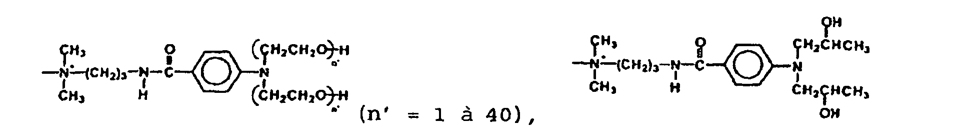

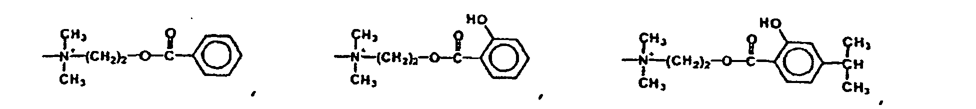

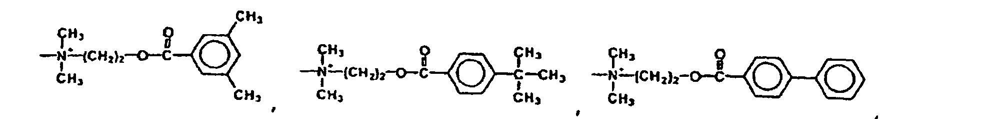

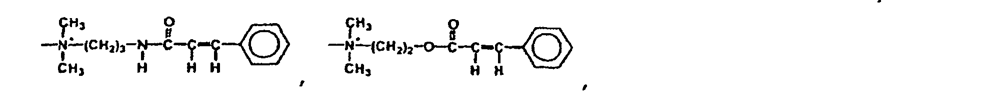

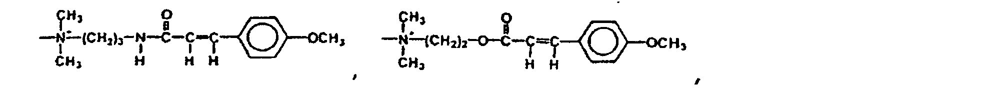

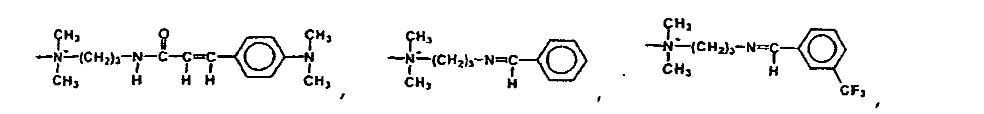

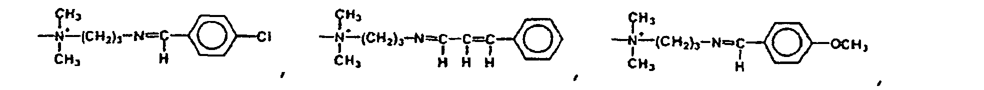

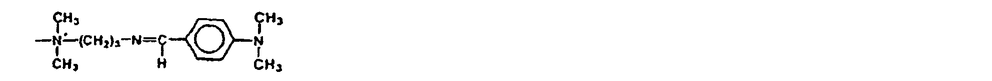

5. Composés absorbant la lumière UV selon les revendications 1 à 4, **caractérisés en ce que** A⁻ est identique ou différent et représente un des radicaux, choisi dans le groupe constitué par les ions acétate, chlorure, bromure, hydrogénosulfate, sulfate, méthosulfate, éthosulfate, citrate, tartrate et lactate, ainsi que les anions d'acides aromatiques, tels que les anions de l'acide p-toluènesulfonique, l'acide benzoïque, l'acide salicylique, l'acide cinnamique, l'acide 4-méthoxycinnamique, l'acide 4-aminobenzoïque, l'acide 4-bis(hydroxypropyl)aminobenzoïque, l'acide 4-bis(polyéthoxy)aminobenzoïque, l'acide 4-diméthylaminobenzoïque, l'acide 3-imidazol-4-yl-acrylique, l'acide 2-phénylbenzimidazole-5-sulfonique, l'acide 3,3'-(1,4-phénylènediméthine)-bis(7,7-diméthyl-2-oxo-bicyclo[2.2.1]heptane-1-méthanesulfonique), l'acide 2-hydroxy-4-méthoxybenzophénone-5-sulfonique et la 3-(4'-sulfo)benzylidène-bornan-2-one.

6. Composé absorbant la lumière UV de formule

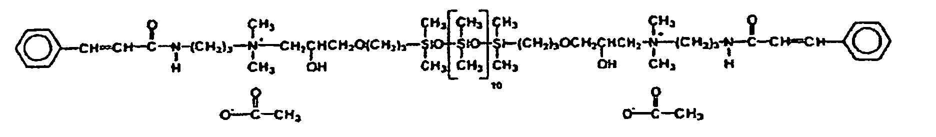

7. Composé absorbant la lumière UV de formule

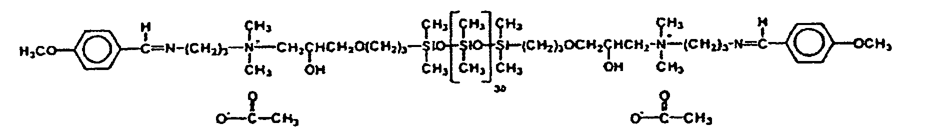

8. Composé absorbant la lumière UV de formule

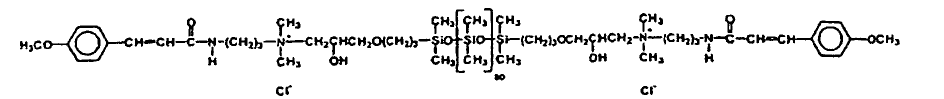

9. Composé absorbant la lumière UV de formule

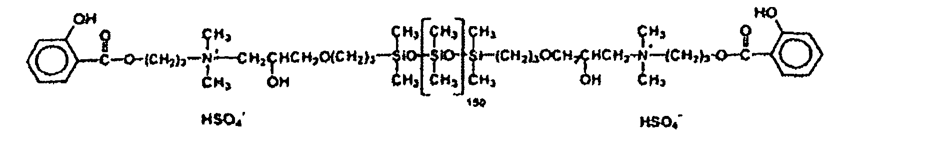

10. Composé absorbant la lumière UV de formule

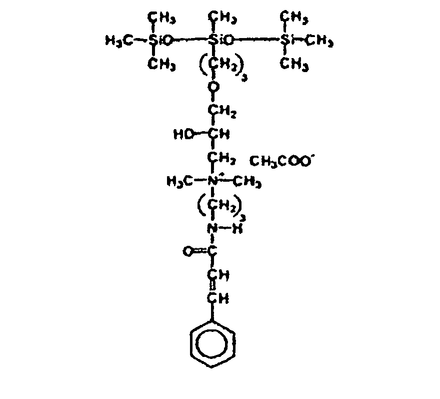

11. Composé absorbant la lumière UV de formule

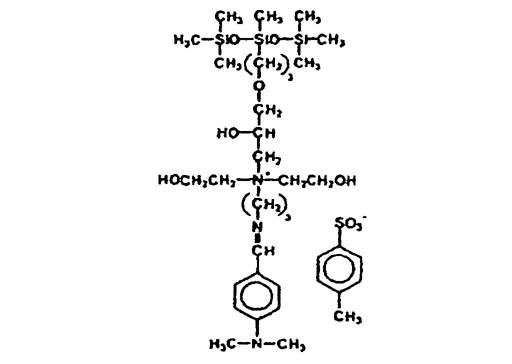

12. Composé absorbant la lumière UV de formule

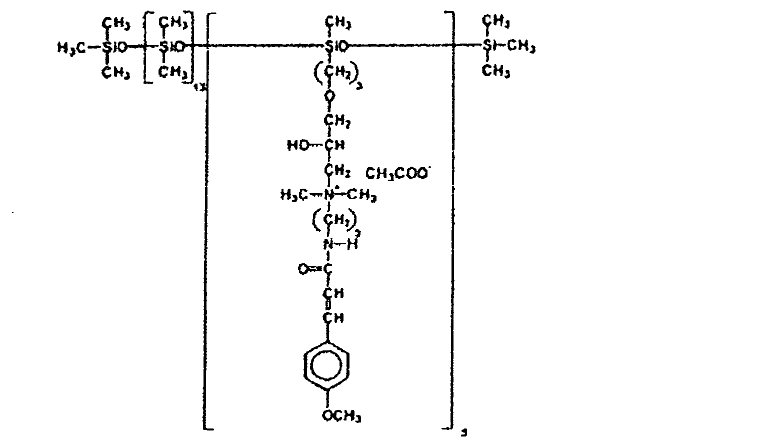

13. Utilisation des composés de formule générale (I) pour la préparation de formulations absorbant la lumière UV.

14. Utilisation des composés de formule générale (I) pour la préparation de formulations cosmétiques absorbant la lumière UV.

15. Utilisation des composés de formule générale (I) pour la préparation d'assouplissants pour le linge absorbant la lumière UV.

16. Utilisation des composés de formule générale (I) pour la préparation d'agents de nettoyage et de soin pour cheveux absorbant la lumière UV.

17. Utilisation des composés de formule générale (I) pour la préparation d'agents de protection, de nettoyage et/ou de soin de la peau absorbant la lumière UV.

18. Procédé pour la préparation de composés absorbant la lumière UV, **caractérisé en ce qu'**on transforme de manière connue en soi des composés de formule générale (II) 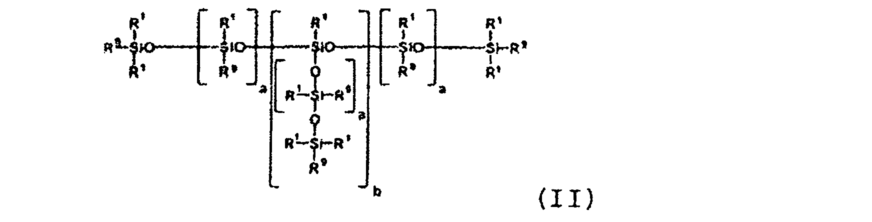 dans laquelle
les radicaux R¹ sont identiques ou différents et signifient à chaque fois des radicaux alkyle inférieurs comprenant 1 à 4 atomes de carbone ou des radicaux phényle,
les radicaux R⁹ peuvent présenter en partie la signification des radicaux R¹ et les autres radicaux R⁹ sont des radicaux monovalents, qui correspondent à la structure du radical M, le radical R⁹ présentant, au lieu de la liaison avec Z et du groupement hydroxyle, un groupement époxyde,
à condition que dans la molécule moyenne, au moins un radical R⁹ est un radical monovalent qui correspond à la structure du radical M, le radical R⁹ présentant, au lieu de la liaison avec Z et du groupement hydroxyle, un groupement époxyde,
a présente, indépendamment l'un de l'autre, une valeur de 1 à 200 et
b présente une valeur de 0 à 10,
avec des amines tertiaires de formule générale (IIb) 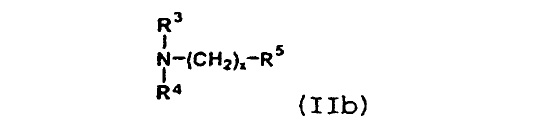 dans laquelle
les radicaux R³, R⁴ représentent des radicaux alkyle comprenant 1 à 22 atomes de carbone ou des radicaux alcényle comprenant 2 à 22 atomes de carbone, où
les radicaux alkyle ou alcényle peuvent présenter des groupements hydroxyle,
R⁵ représente un radical chromophore monovalent, responsable de l'absorption des UV de formule (Ic) 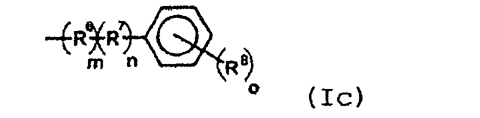 dans laquelle
R⁶ représente 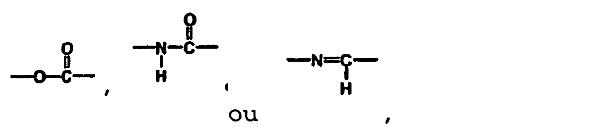 R⁷ représente -CH=CH-,
les radicaux R⁸ peuvent être identiques ou différents et signifient à chaque fois hydrogène,
des radicaux alkyle, halogénoalkyle, halogène, phényle, hydroxyle, alcoxy, amino, alkylamino, dialkylamino, di(hydroxyalkyl)amino ou di(polyalcoxy)amino,
m = 0 ou 1,
n = 0 ou 1,
o = 0 à 5,
x = 0 à 6,
en des rapports de quantités tels qu'au moins un groupement amino tertiaire correspond à chaque groupement époxyde, et on réalise la transformation en présence d'un équivalent, par rapport à l'atome d'azote quaternisé, d'acide HA usuel organique ou inorganique, physiologiquement acceptable, et à des températures de 40 à 120°C.
